# EUROPEAN PATENT APPLICATION

(11) **EP 4 733 301 A1**
(43) Date of publication of application: **29.04.2026**
(21) Application number: 24830745.6
(22) Date of filing: 25.06.2024
(51) Int. Cl.: C07D 405/12, C07D 217/12, C07D 213/02, A61K 47/54, A61K 49/00, A61K 41/00, A61P 35/00

(54) **RADIATION-ACTIVATABLE COMPOUND AND USE THEREOF**

(30) Priority: 26.06.2023 WO PCT/CN2023/102494
(71) Applicant: Peking University, Beijing 100871 (CN)
(72) Inventor: LIU, Zhibo, Beijing 100871 (CN); FU, Qunfeng, Beijing 100190 (CN); GU, Zhi, Beijing 100190 (CN); SHEN, Siyong, Beijing 100190 (CN)
(74) Representative: J A Kemp LLP
(86) International application number: PCT/CN2024/101253
(87) International publication number: WO 2025/002105

(57) **Abstract**

The present invention provides radiation-activatable compounds, drug conjugates thereof and uses thereof. The present invention also provides use of the compound as linking group for drug conjugates.

## Description

### FIELD OF THE INVENTION

The present invention relates to the field of biomedicine, specifically to radiation-activatable compounds, drug conjugates thereof, and uses thereof in treating or diagnosing diseases, for example, in treating or diagnosing cancer.

### BACKGROUND OF THE INVENTION

Over 50% of cancer cases require radiotherapy (or "radiation"), using ionizing radiation to kill cancer cells. Ionizing radiation, including X-rays and γ-rays, has high tissue penetration and low scatter. Modern radiotherapy techniques enable tumor targeting with high spatiotemporal resolution, delivering high radiation doses to tumors while causing minimal damage to surrounding healthy tissues. The high clinical relevance, high tissue penetration, and high tumor targeting characteristics of radiotherapy make X-rays (or γ-rays) the optimal exogenous stimulus for controlled drug release strategies.

However, although strategies using radiotherapy to activate prodrugs were reported in 1990s, their application in tumors were limited by efficiency or chemical selectivity. In tumors, radiotherapy primarily ionizes water rather than the prodrug molecules due to the significant difference in their concentrations. The main products of water radiolysis are hydroxyl radicals (·OH), hydrogen radicals (·H), and hydrated electrons (e⁻_{aq}).

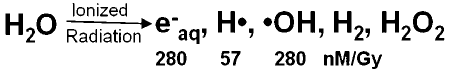

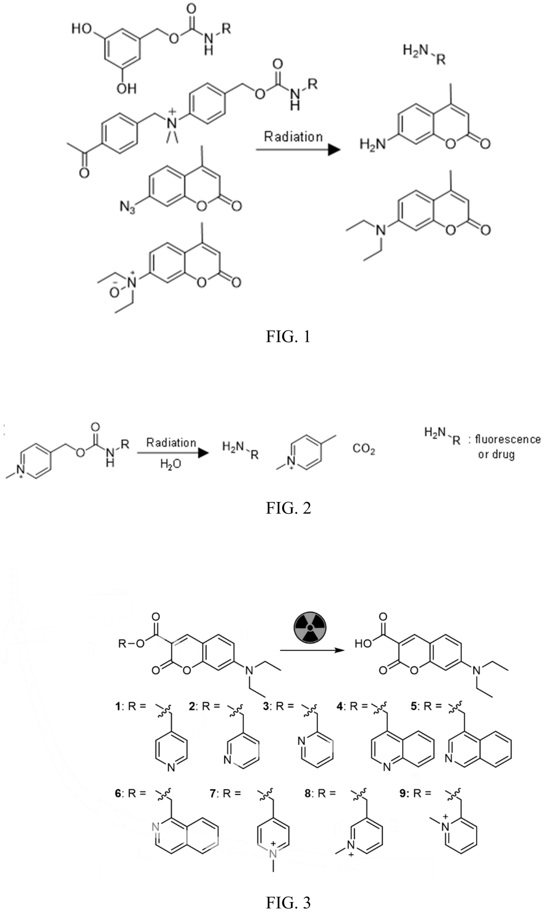

In recent years, a series of radiation-removable protecting groups, mediated by ·OH, ·H or e⁻_{aq}, have been developed (FIG. 1) and are used for prodrug activation in tumors. Notably, the strategy of radiotherapy-activated prodrugs has been rapidly applied in drug development across different fields, including PROTACs, drug conjugates, etc.

Radiation-induced prodrug activation is an ideal method to reduce systemic toxicity of chemotherapy in cancer treatment. Despite many efforts had been made, maintaining the stability of the prodrug before radiation while improving the efficiency and selectivity of radiation activation remains an unresolved challenge.

### SUMMARY OF THE INVENTION

Through persistent efforts, the inventors of the present invention have creatively developed a radiation-removable protecting group (RPG) that is stable before radiation and has high radiation activation efficiency, and have provided a new strategy for effective and rational design of radiation-activatable prodrugs.

Specifically, the present invention provides radiation-activated compounds, drug conjugates thereof and uses thereof. The compound or drug conjugate is activated via radiation to achieve high-efficiency, high-selectivity controlled release, thus obtaining a new class of RPGs.

In one aspect, the present invention provides radiation-activatable compounds, use thereof as linking group for drug conjugates ("ADCs"), and corresponding drug conjugates, whrein the compound or the drug conjugate can be cleaved by radiation to release a drug or fluorescent molecule at the tumor site, thereby providing a potential pathway for developing drugs that produce diagnostic or therapeutic effects through radiation activation.

Further, the present invention provides radiation-activatable compounds, use thereof as linking group for drug conjugates, and corresponding drug conjugates, wherein the compound or the drug conjugate exhibits good stability and high reactivity in biological systems, and its excellent performance in antitumor therapy also provides new insights into concurrent chemoradiotherapy.

Further, the present invention provides radiation-activatable compounds and corresponding drug conjugates thereof, which exhibit strong reactivity under physiological conditions.

In another aspect, the present invention also provides a method for activating the compound or drug conjugate with radiation.

In yet another aspect, the present invention also provides a method for treating or diagnosing a disease, the method comprising administering to an individual the radiation-activatable compound of the present invention or the drug conjugate of the present invention.

In a further aspect, the present invention also provides use of the compound or the drug conjugate in preparing a medicament for treating or diagnosing a disease in an individual, wherein the individual is also irradiated.

In yet another aspect, the present invention also provides use of the compound or the drug conjugate in preparing a combination medicament for treating or diagnosing a disease in an individual, the combination medicament comprising the compound or drug conjugate and radiation.

In one aspect, the present invention provides a radiation-activatable compound, or a pharmaceutically acceptable salt, or solvate, or stereoisomer thereof, the compound having a structure of formula (I) or formula (II): 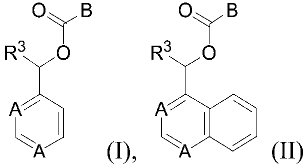 characterized in that:
A is CR¹, N or NR², and only one A in Formula (I) is N or NR², only one A in Formula (II) is N or NR², the carbon atom in the aromatic ring in Formula (I) or Formula (II) is optionally substituted with R¹, wherein R¹ is selected from the group consisting of -H and electron-withdrawing groups, preferably selected from the group consisting of -H, -NO₂, -CN, and halogen, more preferably selected from the group consisting of -H, -NO₂, -CN, -F;
when A is NR², R² is alkyl, preferably C₁₋₆ alkyl, more preferably C₁₋₃ alkyl; R² is optionally substituted with a linking group comprising one or more groups that are or are derived from the group consisting of amino, amido, heterocyclyl, alkyl, alkenyl, alkynyl, polyethylene glycol (PEG), amino acid;
B is any therapeutic agent or chromogenic agent capable of coupling with the aryl ester group moiety of the compound, provided that it can be separated from the aryl ester group moiety under radiation action and release a molecule with therapeutic activity or chromogenic property, for example B is a fluorescent group, or a drug group, preferably a drug group for treating cancer, and more preferably a chemotherapeutic drug group;
R³ is selected from the group consisting of -H and electron-donating groups, preferably selected from the group consisting of -H, alkyl and aryl, more preferably selected from the group consisting of -H, C₁₋₆ alkyl and C₆₋₁₀ aryl, wherein said alkyl and aryl are optionally substituted.

In the compound of Formula (I) or Formula (II), the moiety other than the moiety B (the aryl ester moiety) is a radiation-responsive group, i.e., preferably in the simultaneous presence with water, after radiation, the aryl ester group in the compound of Formula (I) or Formula(II) can be removed, thereby releasing B.

Preferred embodiments of the invention relate to the radiation-activatable compound, or a pharmaceutically acceptable salt, or solvate, or stereoisomer thereof, the compound has a structure of one of the following formulas: 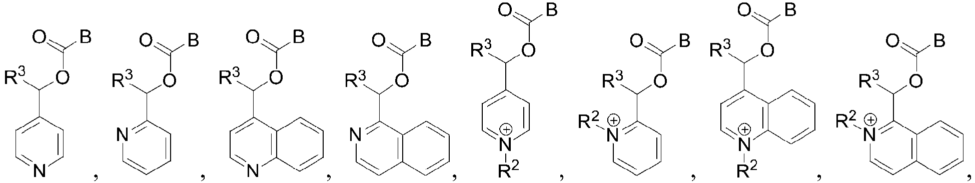
wherein carbon atoms in the aromatic ring are optionally substituted with R¹; R¹, R², R³ and B have meanings as previously described,
preferably in the simultaneous presence with water, after radiation, the aryl ester group in the compound can be removed, thereby releasing B.

In the compound of Formula (I) or Formula (II), B can be any chromogenic or therapeutic agent capable of coupling with the above-described aryl ester group moiety, provided that it can be separated from the aryl ester group moiety under radiation action and releases a molecule with therapeutic activity or chromogenic property. In the present disclosure, the released molecule with therapeutic activity or chromogenic property is usually structurally identical to B in the compound of Formula (I) or Formula (II). In some embodiments, the released molecule with therapeutic activity or chromogenic property is slightly different in structure from B in the compound of Formula (I) or Formula (II), that is, the therapeutic or chromogenic molecule undergoes a chemical reaction under radiation but does not significantly affect its therapeutic activity or chromogenic performance.

Suitable molecules usable as moiety B include, but are not limited to, polypeptides, oligopeptides, peptidomimetics, amino acids, enzyme inhibitors, hormones, toxins, antibiotics, anti-inflammatory substances, fluorescent molecules, and the like.

In a preferred embodiment, the molecule corresponding to the moiety B is a therapeutic agent for treating cancer.

In one embodiment, the present disclosure uses drugs containing a primary or secondary amine as the molecule corresponding to moiety B, wherein moiety B is linked to the aryl ester moiety (radiation-responsive group) through the optionally substituted N atom. In a preferred embodiment, the present disclosure uses an anticancer drug containing a primary or secondary amine as the molecule corresponding to moiety B. Drugs containing primary or secondary amines include, for example, ibrutinib, acalabrutinib, zanubrutinib, doxorubicin, mitomycin-C, mitomycin-A, daunorubicin, aminopterin, actinomycin, bleomycin, 9-aminocamptothecin, N8-acetylspermidine, 1-(2-chloroethyl)-1,2-bis(methylsulfonyl)acylhydrazine, yunnanmycin, gemcitabine, cytarabine, dolastatin, dacarbazine, 5-fluorouracil, and derivatives thereof. Drugs containing a primary or secondary amine also include amino derivatives of drugs that do not naturally comprise an amino group. In other words, a drug that does not originally comprise an amino group can be chemically modified to have an amino group, and then coupled with the radiation-responsive group via the primary or secondary amine coupling method described in the present disclosure.

In one embodiment, the present disclosure uses drugs containing a hydroxyl group as the molecule corresponding to moiety B, wherein moiety B is linked to the aryl ester moiety (radiation-responsive group) through the O atom. In a preferred embodiment, the present disclosure uses anticancer drugs containing a hydroxyl group as the molecule corresponding to moiety B. Hydroxyl containing therapeutic agents include, for example, paclitaxel, docetaxel, gemcitabine, cytarabine, and the like. Molecules containing a hydroxyl group also include hydroxyl derivatives of drugs that do not naturally comprise a hydroxyl group. In other words, a drug that does not originally comprise a hydroxyl group can be chemically modified to have a hydroxyl group, and then coupled with the radiation-responsive group via the hydroxyl coupling method described in the present disclosure.

In one embodiment, the present disclosure uses drugs containing a thiol group as the molecule corresponding to moiety B, wherein moiety B can be linked to the aryl ester moiety (radiation-responsive group) through the S atom. In a preferred embodiment, the present disclosure uses anticancer drugs containing a thiol group as the molecule corresponding to moiety B. Molecules corresponding to moiety B containing a thiol group include, for example, 6-mercaptopurine, etc. Molecules containing a thiol group also include thiol derivatives of drugs that do not naturally comprise a thiol group. In other words, a drug that does not originally comprise a thiol group can be chemically modified to have a thiol group, and then coupled with the radiation-responsive group via the thiol coupling method described in the present disclosure.

Preferred embodiments of the invention relate to the radiation-activatable compound as described above or a pharmaceutically acceptable salt, or solvate, or stereoisomer thereof, wherein
R¹ are all -H,
R², if present, is methyl, optionally substituted with a linking group, and/or
R³ is -H,
the linking group has the meanings as previously described.

Preferred embodiments of the invention relate to the radiation-activatable compound as described above or a pharmaceutically acceptable salt, or solvate, or stereoisomer thereof, wherein
one or more R¹ in Formula (I) or Formula (II) is selected from the group consisting of -NO₂, -CN, and -F,
R², if present, is methyl, and/or
R³ is -H.

Preferred embodiments of the invention relate to the radiation-activatable compound as described above or a pharmaceutically acceptable salt, or solvate, or stereoisomer thereof, wherein
R¹ are all -H,
R², if present, is methyl, and/or
R³ is C₁₋₃ alkyl or phenyl.

In a preferred embodiment, the present invention relates to the radiation-activatable compound as described above or a pharmaceutically acceptable salt, or solvate, or stereoisomer thereof, wherein said B is coumarin and derivatives thereof, rhodamine and derivatives thereof, or auristatin and derivatives thereof:
Preferred embodiments of the invention relate to the radiation-activatable compound as described above or a pharmaceutically acceptable salt, or solvate, or stereoisomer thereof, said B is selected from the group consisting of the following structures: 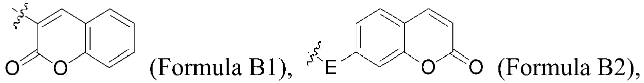 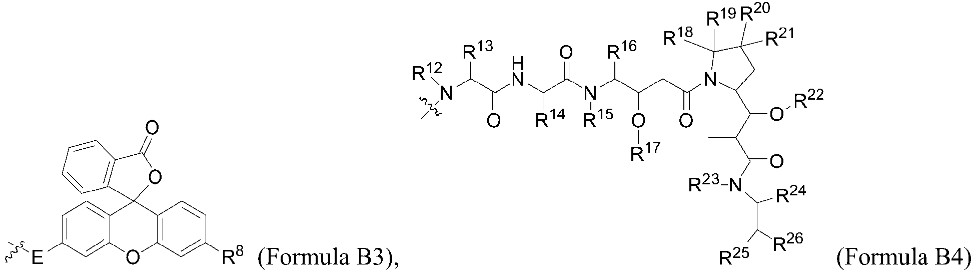
wherein 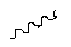 indicates the site where said B attached to the ester group in Formula (I) or Formula (II);
the ring carbon atoms in the Formula (B1), Formula (B2) and Formula (B3) can be optionally substituted by -R⁵, -NR⁶R⁷;
E is an NR⁴ group or O, preferably an NR⁴ group;
R⁸ is NR⁹R¹⁰ or OR⁹;
when said B is auristatin and derivatives thereof, said B is attached to to the ester group in formula (I) or (II) via NR⁴;
R⁴ is -H or alkyl, preferably -H or C₁₋₆ alkyl, more preferably -H or C₁₋₄ alkyl;
each of R⁵, R⁶, R⁷, R⁹ and R¹⁰ is independently selected from H and alkyl, and the alkyl is preferably C₁₋₆ alkyl, more preferably C₁₋₄ alkyl;
in Formula (B4), each of R¹² to R²³ is independently selected from H and alkyl, and the alkyl is preferably C₁₋₆ alkyl, more preferably C₁₋₄ alkyl;
R²⁴ is selected from H, hydroxyl, carboxyl, alkyl, alkoxy, halogen, aryl or heteroaryl, the aryl is preferably C₆₋₁₀ aryl, and the heteroaryl is preferably C₅₋₁₀ heteroaryl;
R²⁵ is selected from H, hydroxyl, alkyl, alkoxy, and halogen;
R²⁶ is selected from aryl or heteroaryl, the aryl or heteroaryl is optionally further substituted by a substituent selected from H, halogen, hydroxy, alkyl, and alkoxy, the aryl is preferably C₆₋₁₀ aryl, and the heteroaryl is preferably C₅₋₁₀ heteroaryl.

Preferred embodiments of the invention relate to the radiation-activatable compound as described above or a pharmaceutically acceptable salt, or solvate, or stereoisomer thereof, said B is selected from the group consisting of the following structures: 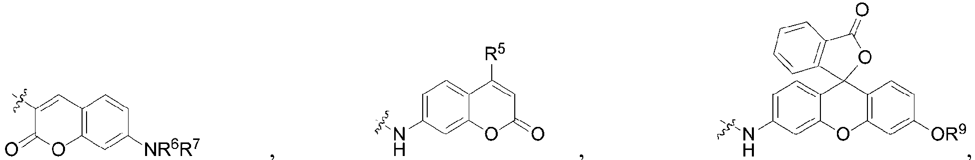 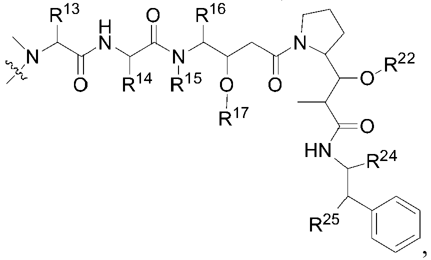
wherein each of R⁵, R⁶, R⁷, R⁹, R¹³ to R¹⁷, and R²² is independently C₁₋₆ alkyl, more preferably C₁₋₄ alkyl, more preferably R⁶ and R⁷ are ethyl, R⁵ and R⁹ are methyl;
R²⁴ is selected from H, hydroxyl, carboxyl, C₁₋₆ alkyl, C₁₋₆ alkoxy, halogen, C₆₋₁₀ aryl and C₅₋₁₀ heteroaryl;
R²⁵ is selected from H, hydroxy, and C₁₋₆ alkyl;

Preferred embodiments of the invention relate to the radiation-activatable compound, or a pharmaceutically acceptable salt, or solvate, or stereoisomer thereof, the radiation-activatable compound is selected from the group consisting of the following structures: 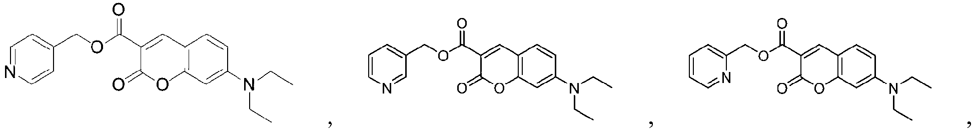 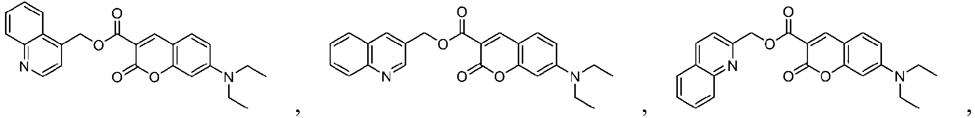 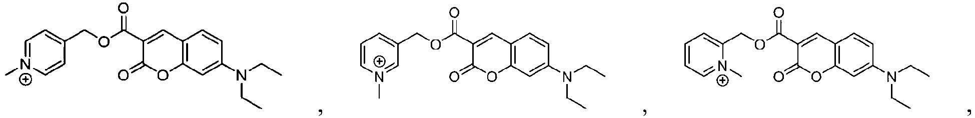 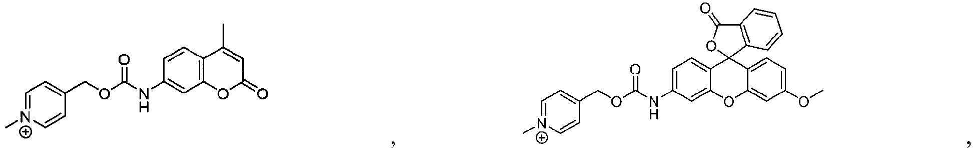 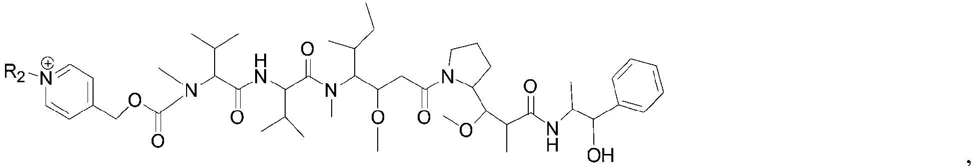 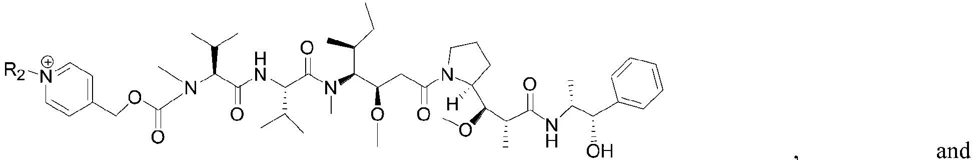 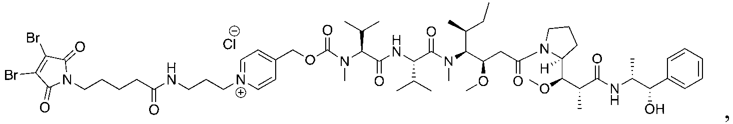 wherein R² is C₁₋₆ alkyl.

In another aspect, the present invention relates to use of the radiation-activatable compound as described above or a pharmaceutically acceptable salt, or solvate, or stereoisomer thereof in preparing a drug conjugate, characterized in that, in Formula (I) or Formula (II), only one A is NR², R² is alkyl, preferably C₁₋₆ alkyl, more preferably C₁₋₃ alkyl; R² is optionally substituted by a linking group comprising one or more groups that are or are derived from amino, amido, heterocyclyl, alkyl, alkenyl, alkynyl, polyethylene glycol (PEG), amino acid.

In a preferred embodiment, the present invention relates to the use as previously described, wherein a drug moiety is attached to Formula (I) or Formula (II) via R², or by reacting with R², thereby preparing the drug conjugate.

In another aspect, the present invention relates to a drug conjugate, or a pharmaceutically acceptable salt, or solvate, or stereoisomer thereof, the drug conjugate comprises a targeting moiety, a linking moiety and a drug moiety, wherein the linking moiety couples the targeting moiety and the drug moiety, and wherein the linking moiety comprises a structure of Formula (I') or Formula (II'): 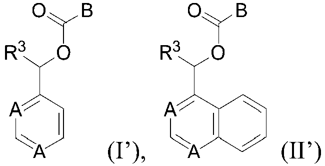 characterized in that:
A is CR¹ or NR^{2'}, and only one A in Formula (I') is NR^{2'}, only one A in Formula (II') is NR^{2'}, the carbon atom in the aromatic ring in Formula (I') or Formula (II') is optionally substituted with R¹, wherein R¹ is selected from the group consisting of -H and electron-withdrawing groups, preferably selected from the group consisting of -H, -NO₂, -CN, and halogen, more preferably selected from the group consisting of -H, -NO₂, -CN, and -F;
R^{2'} is alkyl, preferably C₁₋₆ alkyl, more preferably C₁₋₃ alkyl; R^{2'} is substituted with a linking group attached to the targeting moiety;
B is a fluorescent group, or a drug group, preferably a chemotherapeutic drug group;
R³ is selected from the group consisting of -H and electron-donating groups, preferably selected from the group consisting of -H, alkyl and aryl, more preferably selected from the group consisting of -H, C₁₋₆ alkyl and C₆₋₁₀ aryl, and the alkyl and aryl are optionally substituted;
the targeting moiety is an antibody.

In a preferred embodiment, the present invention relates to the drug conjugate as described above, or a pharmaceutically acceptable salt, or solvate, or stereoisomer thereof, the structure of Formula (I') or Formula (II') having a structure of one of the following formulas: 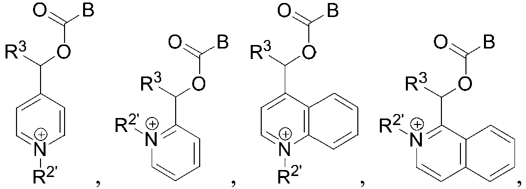 wherein carbon atoms in the aromatic ring are optionally substituted with R¹; R¹, R^{2'}, R³ and B have meanings as previously 1 described.

In a preferred embodiment, the present invention relates to the drug conjugate as described above, or a pharmaceutically acceptable salt, or solvate, or stereoisomer thereof, wherein
R¹ are all -H, and/or
R³ is -H.

In a preferred embodiment, the present invention relates to the drug conjugate, or a pharmaceutically acceptable salt, or solvate, or stereoisomer thereof, wherein
one or more R¹ in Formula (I) or Formula (II) is selected from the group consisting of -NO₂, -CN, and -F, and/or
R³ is -H.

In a preferred embodiment, the present invention relates to a drug conjugate as described above, or a pharmaceutically acceptable salt, or solvate, or stereoisomer thereof, wherein
R¹ are all -H, and/or
R³ is C₁₋₃ alkyl or phenyl.

In a preferred embodiment, the present invention relates to a drug conjugate as described above, or a pharmaceutically acceptable salt, or solvate thereof, or stereoisomer thereof, wherein the linking group comprises one or more groups that are or are derived from amino, amido, heterocyclyl, alkyl, alkenyl, alkynyl, polyethylene glycol (PEG), amino acid.

In yet another aspect, the present invention relates to a method for activating a radiation-activatable compound or a drug conjugate with radiation, characterized in that the radiation-activatable compound or drug conjugate is the compound or drug conjugate as described above, or a pharmaceutically acceptable salt, or solvate, or stereoisomer thereof.

In a preferred embodiment of the present invention, in the method, the radiation is a high-energy radiation ray, preferably X-rays or gamma rays, more preferably the intensity of the X-rays or gamma rays is 60 Gy or less, 50 Gy or less, 40 Gy or less, 30 Gy or less, 20 Gy or less, 10 Gy or less, 8 Gy or less, 6 Gy or less, and 4 Gy or less.

In yet another aspect, the present invention relates to a method for treating or diagnosing a disease, the method comprising administering to an individual a radiation-activatable compound or a drug conjugate, and irradiating the individual with a therapeutically effective amount of radiation, the radiation-activatable compound or the drug conjugate is the compound or the drug conjugate as described above, or a pharmaceutically acceptable salt, or solvate, or stereoisomer thereof.

In a preferred embodiment of the present invention, the method is used for treating or inhibiting cancer, reducing its severity, reducing its risk, or inhibiting its metastasis in an individual.

In a preferred embodiment of the present invention, the cancer is selected from breast cancer, colorectal cancer, lung cancer and gastric cancer.

In a preferred embodiment of the invention, the individual is irradiated after administration of the compound or the drug conjugate, preferably 24 hours, 48 hours, 96 hours, and/or 144 hours after administration of the compound or the drug conjugate.

In a preferred embodiment of the present invention, the compound or the drug conjugate is administered in an amount from about 0.005 mg/kg to about 100 mg/kg, for example, a dose of about 0.005 mg/kg, 0.05 mg/kg, 0.5 mg/kg, 5 mg/kg, 10 mg/kg, 20 mg/kg, 30 mg/kg, 40 mg/kg, 50 mg/kg, 100 mg/kg.

In a preferred embodiment of the present invention, the compound or the drug conjugate, and the radiation are administered once per week, once every two weeks, once every three weeks, or once every four weeks, and the compound or the drug conjugate, and radiation are administered continuously for at least 1 round, at least 2 rounds, at least 3 rounds, at least 4 rounds, at least 5 rounds, at least 6 rounds, at least 7 rounds, at least 8 rounds, at least 9 rounds, at least 10 rounds.

In a preferred embodiment of the present invention, the compound or the drug conjugate is administered parenterally, such as administered intravenously.

In a preferred embodiment of the present invention, the radiation is a high-energy radiation ray, preferably X-rays or gamma rays, more preferably the intensity of the X-rays or gamma rays is 60 Gy or less, 50 Gy or less, 40 Gy or less, 30 Gy or less, 20 Gy or less, 10 Gy or less, 8 Gy or less, 6 Gy or less, and 4 Gy or less.

Preferably, the radiation-activatable compound of the present invention has a structure selected from the following:

| No. | Chemical Formula | Compound Name |
|---|---|---|
| Compound 1 | 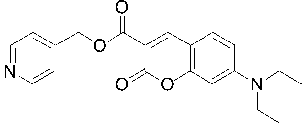 | Pyridin-4-ylmethyl-7-(diethylamino)-2-oxo-2H-chromene-3-carboxylate |
| Compound 2 | 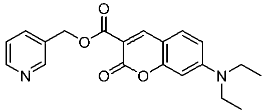 | Pyridin-3-ylmethyl-7-(diethylamino)-2-oxo-2H-chromene-3-carboxylate |
| Compound 3 | 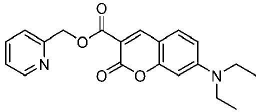 | Pyridin-2-ylmethyl-7-(diethylamino)-2-oxo-2H-chromene-3-carboxylate |
| Compound 4 | 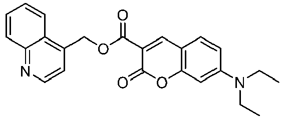 | Quinolin-4-ylmethyl-7-(diethylamino)-2-oxo-2H-chromene-3-carboxylate |
| Compound 5 | 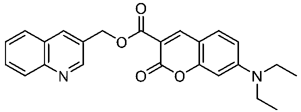 | Quinolin-3-ylmethyl-7-(diethylamino)-2-oxo-2H-chromene-3-carboxylate |
| Compound 6 | 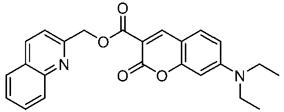 | Quinolin-2-ylmethyl-7-(diethylamino)-2-oxo-2H-chromene-3-carboxylate |
| Compound 7 | 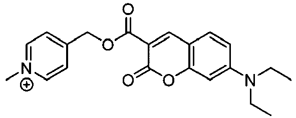 | (4-((7-(diethylamino)-2-oxo-2H-chromene-3-carbonyl)oxy)methyl)-1-methylpyridin-1-ium |
| Compound 8 | 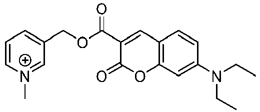 | (3-((7-(diethylamino)-2-oxo-2H-chromene-3-carbonyl)oxy)methyl)-1-methylpyridin-1-ium |
| Compound 9 | 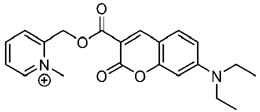 | (2-((7-(diethylamino)-2-oxo-2H-chromene-3-carbonyl)oxy)methyl)-1-methylpyridin-1-ium |
| Compound 10 | 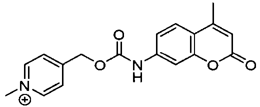 | - |
| Compound NAPC-MeRho | 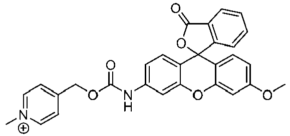 | - |
| Compound VI | 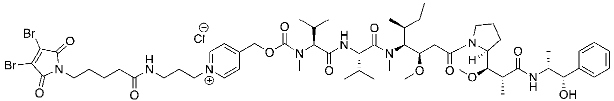 | |

### BRIEF DESCRIPTION OF THE FIGURES

The present invention will be further described below in conjunction with the accompanying drawings.
FIG. 1 shows the structures used in prior art radiation-induced prodrug activation strategies via ·OH or e⁻_{aq}.
FIG. 2 shows that in present invention, the N-alkyl-pyridinium (NAP) group is reduced by e⁻_{aq} generated from water radiolysis, and fluorescent molecules or drugs containing amine (or carboxyl) group can be activated by radiotherapy in tumors in a spatiotemporal manner.
FIG. 3 shows that the pyridyl group or NAP group is identified as a masking group that can be readily removed by e⁻_{aq} generated during water hydrolysis.
FIG. 4 shows the efficacy of Compounds 1-9 in releasing coumarin: 1-9 (10 µM) was irradiated by 60 Gy X-ray in PB (phosphate buffer, pH 7.4, 0.1% dimethyl sulfoxide as co-solvent).
FIG. 5 shows the release efficiency of Compound 7 after adding 10 mM HCOONa or KNO₃ (10µM, in PB, pH 7.4) to the radiation system.
FIG. 6 shows the released energy for capturing electrons (E_{C}) upon reduction of the NAP group and the activation energy for radical bond cleavage (Eₐ), and when E_{C} is smaller, it is easier to bind hydrated electrons, and when activation energy is smaller, it is easier to cleave C-O bond. The calculation results also provide evidence for the efficiency of releasing reporter molecules after irradiation of different groups in experiments.
FIG. 7 shows a schematic diagram of radiation-induced reduction of N-alkyl-pyridinium carbamate (NAPC), which can release the "caged" amino molecule 7-amino-4-methylcoumarin (AMC) remotely.
FIG. 8 shows UPLC spectra of Compound 10 (10 µM, 0.02 M PB, pH 7.4) after receiving a gradient of clinically relevant radiation doses (0-60 Gy), at the temperature of 25 °C.
FIG. 9 shows AMC release versus radiation dose (0-60 Gy).
FIG. 10 shows the chemical structures of other compounds of the prior art.
FIG. 11 respectively shows UPLC spectra of mixtures of Compound 10 with Compound 11 (10 µM 10 + 10 µM 11), Compound 12 (10 µM 10 + 10 µM 12), and Compound 13 (10 µM 10 + 10 µM 13): in PB (10 µM, 0.02M PB, pH 7.4), with or without 60 Gy irradiation at 25 °C.
FIG. 12 respectively shows the competition reaction percentages for mixtures of Compound 10 with Compound 11 (10 µM 10 + 10 µM 11), Compound 12 (10 µM 10 + 10 µM 12), and Compound 13 (10 µM 10 + 10 µM 13).
FIG. 13 shows a schematic diagram of radiation-induced reduction, which releases the "fluorescence-caged" MeRho from carbamate-containing NAPs.
FIG. 14 shows a photography of radiation dose-dependent fluorescence.
FIG. 15 sequentially shows fluorescence emission spectra and UPLC spectra of NAPC-MeRho (10 µM, 0.02M PB, pH 7.4) after treatment with a radiation gradient, determining that NAPC-MeRho produces MeRho in a radiation dose-dependent manner. The detector wavelength was 481 nm, which is the characteristic absorption of MeRho.
FIG. 16 shows confocal fluorescence images. Cells were pretreated with NAPC-MeRho (10 µM, 0.1% DMSO) for 30 minutes under hypoxic condition, then subjected to different amounts of radiation. Scale bar, 50 µm.
FIG. 17 respectively shows fluorescence intensity per single 4T1-FAP cell (left), HT1080-FAP cell (middle), and U87MG cell (right) in the confocal microscopy field of view: 10 cells were randomly selected per experiment (two-tailed unpaired Student's t-test, ***P<0.001).
FIG. 18 shows representative confocal fluorescence images of tumor sections: scale bar, 100 µm.
FIG. 19 shows average fluorescence intensities of tumor sections: five fields of view were randomly selected per experiment (two-tailed unpaired t-test, ***P<0.001), using BALB/c mice subcutaneously implanted with 4T1-FAP cells.
FIG. 20 shows the design of NAPC-ADC conjugate: the NAPC linker is reduced to release the carbamate, which undergoes decarboxylation, returning to the highly toxic MMAE.
FIG. 21 shows the purity of the NAPC-ADC conjugate determined by HIC HPLC (left) and MALDI-TOF analysis (right), respectively.
FIG. 22 shows drug release under a radiation dose gradient: [NPC-ADC] = 100 nM.
FIG. 23 shows cell viability assay of 4T1-FAP cells treated with NAPC-ADC under hypoxic conditions, alone or a dditionally with X-ray.
FIG. 24 shows detection of α-tubulin immunofluorescence via confocal microscopy to determine MMAE produced by NAPC-ADC + X-ray, thereby destabilizing tubulin within the cytoskeleton of 4T1-FAP cells. Scale bar = 25 µm.
FIG. 25 shows that radiotherapy-induced controlled release of NAPC-ADC triggers tumor regression in mice, dynamic positron emission tomography computed tomography images of 4T1-FAP tumor-bearing mice at specified time points after intravenous injection of [⁸⁹Zr]NAPC-ADC.
FIG. 26 shows the time-activity curves (TAC) of [⁸⁹Zr]NAPC-ADC in blood, muscle and tumor.
FIG. 27 shows the treatment regimen for 4T1-FAP tumor-bearing mice: 20 BABL/c mice were subcutaneously implanted with 4T1-FAP cells and randomly divided into 4 groups (n = 5), treated with PBS; NAPC-ADC (5 mg/kg, intravenous injection); radiotherapy as indicated; NAPC-ADC (5 mg/kg) and radiotherapy (4 Gy each time, once every two days).
FIG. 28 shows detection of tumor concentration of MMAE after mice were injected with NAPC-ADC (5 mg/kg) alone or additionally with X-ray (4, 8, 12, and 16 Gy).
FIG. 29 shows the tumor growth curve of tumor-bearing mice after treatment.
FIG. 30 shows the average tumor weight of tumor-bearing mice on day 21 after treatment.
FIG. 31 shows the body weight change curve of mice. (***P<0.001, **P=0.0018, two-tailed unpaired t-test).
FIG. 32 shows the irradiation release efficiency of Compound 1: liquid chromatography characterization data for the release of the coumarin model molecule from Compound 1 at a concentration of 10 µM under 60 Gy irradiation conditions.
FIG. 33 shows irradiation release efficiency tests under different redox conditions: (A) Liquid chromatography profiles of the release of the coumarin model molecule from 10 µM Compound 1 under conditions of 20 Gy irradiation, 20 Gy + 10 mM KNO₃, and 20 Gy + 10 mM HCO₂Na; (B) The coumarin release efficiency was 16.7% under irradiation conditions, 1.3% in potassium nitrate + irradiation, and 27.3% in sodium formate + irradiation.

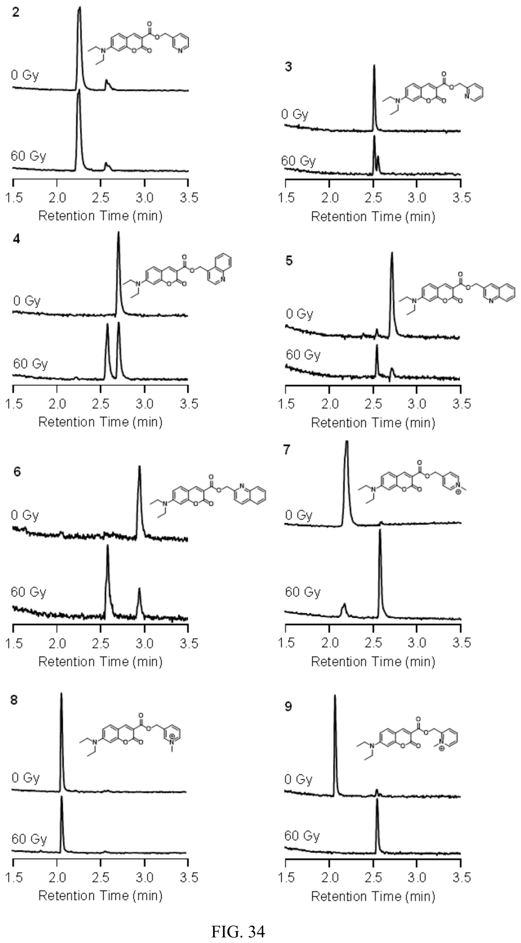

FIG. 34 shows the irradiation release efficiency of Compounds 2-9 (10 µM) under 60 Gy X-ray conditions.
FIG. 35 shows the release of Compound 7 under different irradiation doses: (A) Liquid chromatography profiles at different doses; (B) Statistical data of coumarin release by irradiation at different doses, showing a linear relationship; (C) UV-Vis absorption spectra of Compound 7 and the released carboxycoumarin.
FIG. 36 shows the release efficiency of Compound 7 under different redox conditions: (A) UPLC chromatograms of 10 µM Compound 7 after 20 Gy irradiation, 20 Gy + 10 mM KNO₃, and 20 Gy + 10 mM HCO₂Na; (B) Coumarin release rate was 27.6% under control conditions, 0.0% under oxidative KNO₃, and 53.8% under reductive HCO₂Na.
FIG. 37 shows the release efficiency of Compound 10 under different radiation doses: (A) UPLC-MS chromatograms of increasing release of Compound 10 with increasing irradiation doses (324 nm, characteristic absorption wavelength of Compound 10); (B) UPLC-MS chromatograms of increasing release of Compound 10 with increasing irradiation doses (342 nm, characteristic absorption wavelength of coumarin); (C) Linear relationship between coumarin release concentration and irradiation dose; (D) UV-Vis absorption spectra of Compound 10 (NAPC-AMC) and coumarin (AMC).
FIG. 38 shows characterization of pyridinium irradiation products: (A) 4-[(acetoxy)methyl]-1-methylpyridinium (Intermediate 1) produces 1,4-dimethylpyridinium (Intermediate 2) after irradiation; (B) UPLC-MS chromatogram of 1 mm Intermediate 1 after 5000 Gy X-ray irradiation; (C) Mass spectrum signal of the retention peak for Intermediate 1; (D) Mass spectrum signal of the retention peak for Intermediate 2.

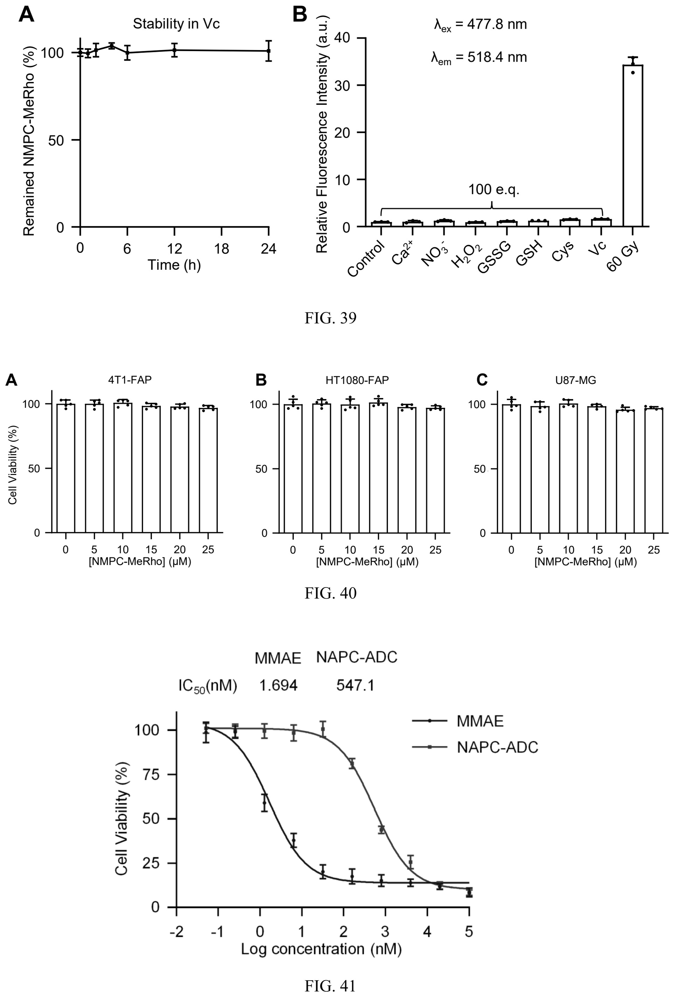

FIG. 39 shows the stability of NAPC-MeRho *in vitro:* (A) NAPC-MeRho (10 µM) treated with 100 e.q VcNa, pH 7.4; (B) Fluorescence intensity of MeRho release under different reducing conditions.
FIG. 40 (A) Cell viability of 4T1-FAP cells treated with 10 µM NAPC-MeRho; (B) Cell viability of HT1080-FAP cells treated with 10 µM NAPC-MeRho; (C) Cell viability of U87-MG cells treated with 10 µM NAPC-MeRho.
FIG. 41 shows the cell viability of 4T1-FAP treated with MMAE and NAPC-ADC: IC₅₀ of MMAE for 4T1-FAP was 1.694 nM; IC₅₀ of NAPC-ADC for 4T1-FAP was 547.1 nM.

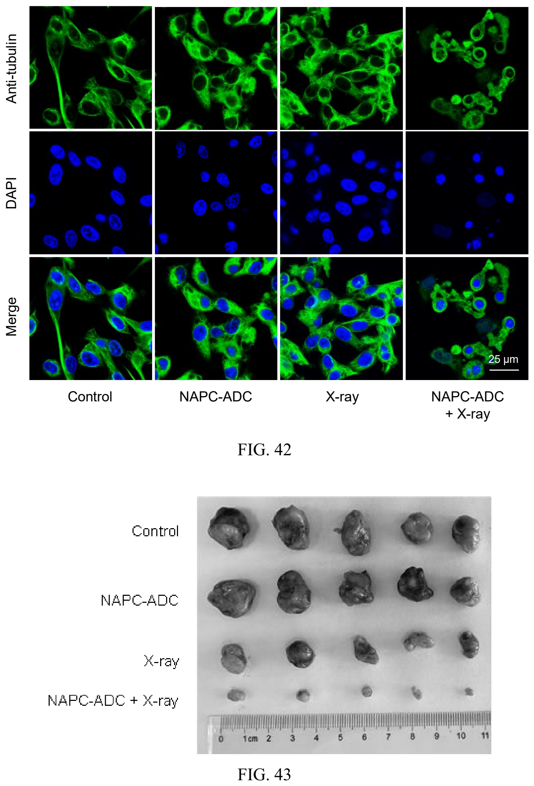

FIG. 42 shows *in vitro* immunofluorescence staining of NAPC-ADC. Detection via confocal microscopy α-tubulin immunofluorescence of MMAE produced by NAPC-ADC + X-ray, destabilizing tubulin within the cytoskeleton of HT1080-FAP cells. Scale bar = 25 µm.
FIG. 43 shows photograph of representative tumors on day 21 after different treatments.

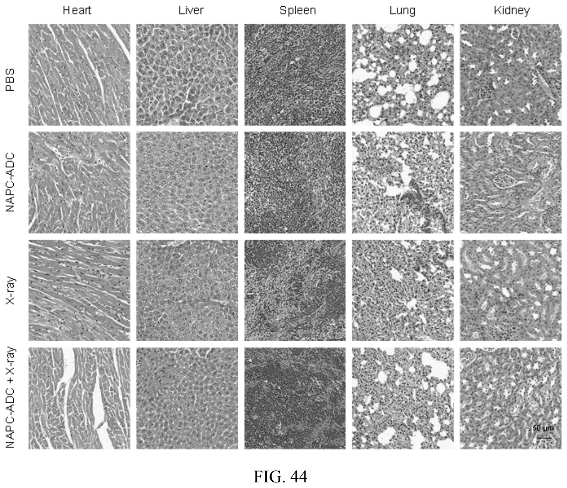

FIG. 44 shows hematoxylin and eosin stained tissue sections of major organs (heart, liver, spleen, lung, kidney) from different treatment groups. No obvious abnormalities were observed in these major organs of mice after treatment.

### DETAILED DESCRIPTION OF THE INVENTION

The present invention provides a radiation-activatable compound, a drug conjugate thereof and uses thereof.

Before further describing the present invention, certain terms used in the description, examples, and appended claims are collected in the sections below. The definitions listed herein should be read and understood by those skilled in the art in light of the remainder of the present disclosure. Unless otherwise defined, all technical and scientific terms used herein have the same meaning as commonly understood by one of ordinary skill in the art to which this invention belongs.

### DEFINITIONS

Unless otherwise indicated, when any type of range is disclosed or claimed, it is intended to separately disclose or claim each possible numerical value that could reasonably be encompassed within that range, including any subranges encompassed therein. For example, a group number of 1 to 6 indicates integers within this range, where 1-6 should be understood to include 1, 2, 3, 4, 5, 6, and also subranges such as 1-5, 1-4, and 1-3.

The description of the present disclosure should be interpreted consistently with the laws and principles of chemical bonds. In some cases, hydrogen atoms may be removed to accommodate substituents at a given position.

The terms "include", "contain", or "comprise" used in this disclosure are intended to mean that the elements preceding these words encompass the elements listed after them and their equivalents, without excluding unrecorded elements. As used herein, the terms "contain" or "include (comprise)" can be open ended, semi-close ended, or close ended. In other words, the terms also encompasss "consisting essentially of ......" or "consisting of ... ...".

The term "pharmaceutically acceptable" in this disclosure means that a compound or composition is chemically and/or toxicologically compatible with other ingredients constituting the formulation and/or with the human or mammal being treated or prevented from the disease or condition.

"Stereoisomer" refers to a compound having the same chemical structure, but different spatial arrangements of atoms or groups. Stereoisomers include enantiomers, diastereomers, conformational isomers (rotational isomer), geometric isomers, (cis/trans) isomers, atropisomers, and the like.

"Radiation" in the context of the present disclosure refers to non-visible or ultraviolet radiation, preferably high-energy radiation, such as preferably X-rays or gamma rays, for example, any radiation used in radiotherapy.

When "about" precedes a value or ratio, it means such value or ratio ±10%, preferably ±5%, more preferably ±1%.

The term "group" refers to a moiety formed by removing one, two, or more hydrogen radicals from a molecule. That is, the "fluorescent group" or "drug group" refers to a group formed by removing one, two or more hydrogen radicals from a fluorescent molecule or a drug molecule commonly used in the art.

The expression "optionally substituted" means that one, two, three, or more than three hydrogen atoms in a group can be substituted independently of each other by various substituents. The substituents may be selected from alkyl, alkenyl, alkoxy, halogen, cyano, amino, nitro, and -OH.

The term "alkyl" refers to a saturated linear or branched carbon chain. Preferably, the chain comprises 1 to 10 carbon atoms, i.e., 1, 2, 3, 4, 5, 6, 7, 8, 9, or 10 carbon atoms, preferably comprises 1 to 6 carbon atoms, most preferably comprises 1 to 3 carbon atoms. The alkyl is, for example, methyl, ethyl, n-propyl, isopropyl, butyl, isobutyl, tert-butyl, pentyl, hexyl, pentyl or octyl. The alkyl is optionally substituted.

The term "alkoxy" includes -O-alkyl groups and alkyl groups where the O atom is within the alkyl chain, e.g., -CH₂-O-CH₃, comprising from 1 to 10 carbon atoms, preferably from 1 to 6 carbon atoms, most preferably from 1 to 3 carbon atoms. The alkoxy is optionally substituted.

The term "alkenyl" includes both linear chain alkyl groups and branched alkyl groups comprising at least two carbon atoms and at least one carbon-carbon double bond, comprising from 2 to 10 carbon atoms, preferably from 2 to 6 carbon atoms, most preferably from 2 to 3 carbon atoms. The alkenyl is optionally substituted.

The term "alkynyl" denotes an alkyl group having at least one site of unsaturation, i.e., a carbon-carbon sp triple bond, comprising 2 to 10 carbon atoms, preferably 2 to 6 carbon atoms, most preferably 2 to 3 carbon atoms. Examples of alkynyl include, but are not limited to, ethynyl (-C=CH), propargyl (-CH₂C=CH), 1-propynyl (-C=C-CH₃), etc. The alkynyl is optionally substituted.

The term "aryl" preferably refers to an aromatic monocycle containing 6 carbon atoms, an aromatic bicyclic system containing 10 carbon atoms, or an aromatic tricyclic system containing 14 carbon atoms. Examples are phenyl, naphthyl, or anthryl. The alkyl is optionally substituted.

The term "heteroaryl" preferably refers to a five- or six-membered aromatic monocycle in which at least one carbon atom is substituted by 1, 2, 3, or 4 (for a five-membered ring) or 1, 2, 3, 4, or 5 (for a six-membered ring) identical or different heteroatoms, the heteroatoms are preferably selected from O, N, and S; an aromatic bicyclic system in which 1, 2, 3, 4, 5, or 6 carbon atoms of 8, 9, 10, 11, or 12 carbon atoms are substituted by identical or different heteroatoms, the heteroatoms are preferably selected from O, N, and S; or an aromatic tricyclic system in which 1, 2, 3, 4, 5, or 6 carbon atoms of 13, 14, 15, 16 carbon atoms are substituted by identical or different heteroatoms, and the heteroatoms are preferably selected from O, N, and S. Nitrogen atoms present in heteroaryl can exist in the form of an onium group, e.g., an alkyl or oxygen attached to a trivalent aromatic N to form an onium group, that is, "heteroaryl" in the context of the present application includes heteroaryl in free form (N atom not salified) and heteroaryl existing as an onium salt. Examples are pyridyl, N-oxidopyridyl, N-alkylpyridyl, oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3-thiadiazolyl, 1,2,5-thiadiazolyl, pyridyl, pyrimidinyl, pyrazinyl, 1,2,3-triazinyl, 1,2,4-triazinyl, 1,3,5-triazinyl, 1-benzofuranyl, 2-benzofuranyl, indolyl, isoindolyl, benzothienyl, 2-benzothienyl, 1H-indazolyl, benzimidazolyl, benzoxazolyl, indoloxazinyl, 2,1-benzoxazolyl, benzothiazolyl, 1,2-benzisothiazolyl, 2,1-benzisothiazolyl, benzotriazolyl, quinolinyl, isoquinolinyl, quinoxalinyl, quinazolinyl, quinolinyl, 1,2,3-benzotriazinyl or 1,2,4-benzotriazinyl.

The term "heterocyclyl" or "heterocycle" refers to a non-aromatic or aromatic 5-, 6-, or 7-membered ring or polycyclic group, including but not limited to bicyclic or tricyclic groups comprising fused six-membered or seven-membered rings, wherein at least one carbon atom in one ring is substituted by a heteroatom. Each heteroatom is independently selected from atoms of oxygen, sulfur (including sulfoxide and sulfone) and/or nitrogen (which may be oxidized or quaternized). The terms "heterocyclyl," "heterocycle" or "heterocyclic" are intended to include groups wherein -CH₂- in the ring is substituted by -C(=O)-, for example cyclic ureido groups (e.g., 2-imidazolidone) and cyclic amide groups (e.g., β-lactam, γ-lactam, δ-lactam, and ε-lactam) and piperazin-2-one; wherein (i) each 5-membered ring has 0 to 2 double bonds, each 6-membered ring has 1 to 3 double bonds, (ii) nitrogen and sulfur heteroatoms may optionally be oxidized, and (iii) nitrogen atoms may optionally be quaternized. Representative heterocycles include, but are not limited to, pyrrolyl, pyrrolidinyl, dioxopyrrolyl, pyrazolinyl, imidazolyl, imidazolidinyl, piperidinyl, piperazinyl, oxazolyl, oxazolidinyl, isoxazolyl, isoxazolidinyl, morpholinyl, thiazolyl, thiazolidinyl, isothiazolidinyl, furanyl, and tetrahydrofuranyl.

The terms "chemotherapeutic agent" or "chemotherapeutic agent" refer to a chemotherapeutic drug that can kill tumor cells, which can act on different stages of tumor cell growth and reproduction, thereby inhibiting or killing tumor cells.

The term "amino acid" refers to any compound containing both an amino group and a carboxylic acid group. Although amino groups most commonly appear adjacent to the carboxyl functional group, the amino group can be located anywhere within the molecule. Amino acids can also contain additional functional groups, such as amino, thio, carboxy, carboxamide, imidazole, and the like. Amino acids can be synthetic or naturally occurring, and can be used in their racemic or optically active (D- or L-) forms.

The term "polyethylene glycol" refers to one or more -CH₂CH₂O- repeating units.

The term "linking group" refers to any chemically suitable linking group. Preferably, the linking group does not break or only breaks slowly under physiological conditions.

The term "antibody" refers to an immunoglobulin molecule or an immunologically active moiety thereof that binds to a specific antigen (e.g., a cancer cell antigen, viral antigen, or microbial antigen). In those embodiments where the targeting moiety is an antibody and the antibody is a full-length immunoglobulin molecule, the antibody comprises two heavy chains and two light chains, each heavy and light chain comprising three complementarity determining regions (CDRs). In those embodiments where the targeting moiety is an antibody and the antibody is an immunologically active moiety of an immunoglobulin molecule, the antibody can be a single domain antibody (dAb), diabody, triabody, tetrabody, and the antibody used as the targeting moiety can be, for example, a natural antibody, synthetic antibody, monoclonal antibody, polyclonal antibody, chimeric antibody, humanized antibody, multispecific antibody, bispecific antibody, dual specific antibody, anti-idiotypic antibody, or a fragment thereof, that retains the ability to bind the specific antigen.

The term "derivative" refers to a compound having the same backbone structure as the parent core structure, where substituents on the backbone may vary, e.g., ring atoms substituted by alkyl, alkenyl, alkoxy, halogen, cyano, amino, alkylamino, nitro, -OH, or substituents on the backbone can be alkyl, alkenyl, alkoxy, halogen, cyano, amino, alkylamino, nitro, -OH.

The term "rhodamine" refers to dye molecules having the following two backbone structures, wherein ring atoms are optionally substituted.

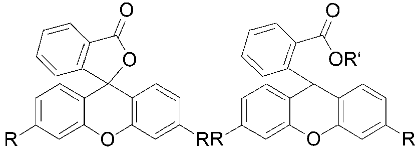

The term "coumarin" refers to dye molecules having the following backbone structure, wherein ring atoms are optionally substituted.

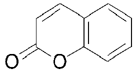

The term "auristatin" refers to drug molecules having the following backbone structure, wherein substituents R¹¹ to R²⁶ on the backbone can each independently be hydrogen, alkyl, alkenyl, alkoxy, halogen, hydroxy (-OH), carboxy (-COOH), aryl, heteroaryl, cyano, amino, alkylamino or nitro.

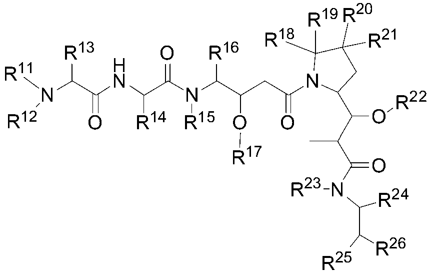

The term "pharmaceutically acceptable salt" refers to relatively non-toxic addition salts of the compounds of the present disclosure. Refer to, for example, S. M. Berge et al., "Pharmaceutical Salts, J. Pharm. Sci. 1977, 66, 1-19.

Suitable pharmaceutically acceptable salts of the compounds of the present disclosure can be, for example, acid addition salts of sufficiently basic compounds of the present disclosure carrying a nitrogen atom in the chain or ring, for example, the acid addition salts prepared with inorganic acids such as hydrochloric acid, hydrobromic acid, hydroiodic acid, sulfuric acid, phosphoric acid or nitric acid, or the acid addition salts prepared with organic acids such as formic acid, acetic acid, acetoacetic acid, pyruvic acid, trifluoroacetic acid, propionic acid, butyric acid, hexanoic acid, heptanoic acid, undecanoic acid, lauric acid, benzoic acid, salicylic acid, 2-(4-hydroxybenzoyl)benzoic acid, camphoric acid, cinnamic acid, cyclopentanepropionic acid, 3-hydroxy-2-naphthoic acid, nicotinic acid, pamoic acid, pectinic acid, persulfuric acid, 3-phenylpropionic acid, picric acid, pivalic acid, 2-hydroxyethanesulfonic acid, itaconic acid, aminosulfonic acid, trifluoromethanesulfonic acid, dodecylsulfuric acid, ethanesulfonic acid, benzenesulfonic acid, p-toluenesulfonic acid, methaylsulfonic acid, 2-naphthalenesulfonic acid, naphthalenedisulfonic acid, camphorsulfonic acid, citric acid, tartaric acid, stearic acid, lactic acid, oxalic acid, malonic acid, succinic acid, malic acid, adipic acid, alginic acid, maleic acid, fumaric acid, D-gluconic acid, mandelic acid, ascorbic acid, glucoheptonic acid, glycerophosphoric acid, aspartic acid, sulfosalicylic acid or thiocyanic acid.

Furthermore, another suitable pharmaceutically acceptable salt of a sufficiently acidic compound of the present invention is an alkali metal salt such as sodium salt or potassium salt, an alkaline earth metal salt such as calcium salt or magnesium salt, ammonium salt, triethylamine salt, or a salt formed with an organic base providing a physiologically acceptable cation, for example, salts formed with the following substances: N-methylglucamine, dimethylglucamine, ethylglucamine, lysine, dicyclohexylamine, 1,6-hexanediamine, ethanolamine, glucosamine, sarcosine, serinol, tris(hydroxymethyl)aminomethane, aminopropanediol, 1-amino-2,3,4-butanetriol. Additionally, basic nitrogen-containing groups can be quaternized with reagents such as lower alkyl halides, e.g., chlorides, bromides, and iodides of methyl, ethyl, propyl, and butyl; dialkyl sulfates, e.g., dimethyl sulfate, diethyl sulfate, dibutyl sulfate, and dipentyl sulfate; long-chain halides such as chlorides, bromides, and iodides of decyl, lauryl, myristyl, and stearyl; aralkyl halides such as bromides of benzyl and phenethyl and the like.

Those skilled in the art will also recognize that acid addition salts of the claimed compounds can be prepared by reacting the compound with an appropriate inorganic or organic acid by any one of various known methods. Alternatively, alkali metal salts and alkaline earth metal salts of acidic compounds of the present disclosure can be prepared by reacting them with an appropriate base by various known methods.

The present invention includes all possible salts of the compounds of the present disclosure, which can be a single salt or any mixture of said salts in any ratio.

The term "solvate" is a substance formed by combining, physically associating and/or solvating a compound of the invention with solvent molecules, for example, a disolvate, monosolvate or hemisolvate, where the ratio of solvent molecules to the compound of the invention is about 2:1, about 1:1 or about 1:2, respectively. This physical association involves ionization and covalent bonding (including hydrogen bonding) to varying degrees. In certain instances (e.g., when one or more solvent molecules are incorporated into the crystal lattice of a crystalline solid), solvates can be isolated. Thus, solvates include solution-phase and isolable solvates. Compounds of the present invention may be in solvated form with pharmaceutically acceptable solvents such as water, methanol, and ethanol, and the present application is intended to encompass both solvated and non-solvated forms of the compounds of the invention. One solvate is a hydrate.

The term "halogen" refers to fluorine, chlorine, bromine, iodine, and astatine.

The term "optional (optionally)" means that the situation may or may not occur.

The term "effective amount" refers to the amount of an active ingredient that will alleviate one or more symptoms of the disease being treated to some extent after administration.

The term "individual" includes human or non-human animals. Exemplary human individuals include a human individual (referred to as a patient) having a disease (e.g., a disease described herein) or a normal individual. "Non-human animals" in the present invention include all vertebrates, such as non-mammals (e.g., birds, amphibians, reptiles) and mammals such as non-human primates, domestic animals and/or domesticated animals (e.g., sheep, dogs, cats, cows, pigs, etc.).

### Examples

### Reagents and Models Used:

Starting materials for the examples are commercially available and/or can be prepared by various methods known to those skilled in the art of organic synthesis. Those skilled in the art of organic synthesis will appropriately select reaction conditions (including solvent, reaction atmosphere, reaction temperature, duration of experiment, and post-treatment) in the synthesis methods described below. Those skilled in the art of organic synthesis will understand that functional groups present on various parts of the molecule should be compatible with the proposed reagents and reactions.

All reagents and compounds for synthesis can be purchased in mainland China (excluding Hong Kong, Taiwan and Macau) through general commercial channels, suppliers include J&K (China), Bide Pharmatech Co., Ltd, Sinopharm Group Co., Ltd., Beijing Chemical Works, Yeasen Biotechnology, Abcam (ab64503).

Cell model: Mouse breast cancer 4T1 cell line (4T1-FAP, WuXi AppTec, China) transfected with fibroblast activation protein was grown in RPMI-1640 (Roswell Park Memorial Institute-1640) medium containing 10% FBS (fetal bovine serum), 1% penicillin/streptomycin, and 2 µg/mL blasticidin S.

Human fibrosarcoma HT1080 cell line (HT1080-FAP, WuXi AppTec, China) transfected with fibroblast activation protein was grown in EME (Eagle's minimum essential medium) medium containing 10% FBS, 1% penicillin/streptomycin, and 4 µg/mL blasticidin S.

The U87MG cell line was from the American Type Culture Collection (ATCC) and grown in DMEM (Dulbecco's modified eagle medium) medium containing 10% FBS and 1% penicillin/streptomycin.

All cell cultures were incubated at 37 °C and 5% carbon dioxide environment.

### Instrument Used:

NMR spectra were recorded on a Bruker AVANCE 400 MHz spectrometer and a Bruker AVANCE 500 MHz spectrometer.

Ultra-performance liquid chromatography-mass spectrometry (UPLC-MS) was performed using an ACQUITY UPLC H-Class PLUS instrument equipped with a Waters PDA eλ detector and a Waters ACQUITY QDA mass spectrometer.

High-resolution mass spectrometry analysis was performed on a Bruker Fourier transform ion cyclotron resonance mass spectrometer.

Absorption spectra were measured with a UV-1100 spectrophotometer.

Confocal fluorescence images were recorded on an A1R-si laser scanning confocal microscope (Nikon, Japan).

PET images were taken on a microPET (Mediso Medical Imaging Systems).

To make the purpose and technical solutions of the present invention clearer, the present invention will be further described below with specific examples. It should be understood that these examples are not intended to limit the scope of the invention. Moreover, specific experimental methods not mentioned in the following examples were performed according to conventional experimental methods.

Compounds 11, 12 and 13 as comparative examples were synthesized according to the following prior art documents:
[1] Sundararajan, C. & Falvey, D. E. C-O Bond Fragmentation of 4-Picolyl- and N-Methyl-4-picolinium Esters Triggered by Photochemical Electron Transfer. J. Org. Chem. 69, 5547-5554, doi:10.1021/jo049501j (2004).
[2] Fu, Q. et al. External-Radiation-Induced Local Hydroxylation Enables Remote Release of Functional Molecules in Tumors. Angew. Chem. Int. Ed. 59, 21546-21552, doi:https://doi.org/10.1002/anie.202005612 (2020).

### Example 1: Synthesis of Compound 1

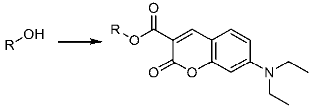

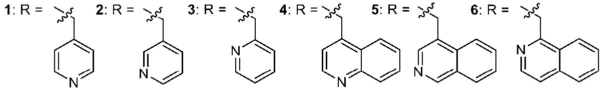

Synthetic Route of Compounds 1 to 6

Pyridin-4-ylmethanol (109 mg, 1.0 mmol, 1.0 eq.), DCC (247 mg, 1.2 mmol, 1.2 eq.) and DMAP (12 mg, 0.1 mmol, 0.1 eq.) were dissolved in DCM (5 mL, anhydrous). 7-(diethylamino)-2-oxo-2H-1-chromene-3-carboxylic acid was added. The reaction mixture was stirred at room temperature for 4 hours, monitored by thin-layer chromatography (TLC). The reaction mixture was then washed with water and saturated sodium chloride solution, extracted with ethyl acetate. The organic layer was dried over Na₂SO₄, evaporated under reduced pressure, and the crude product was purified by flash chromatography eluting with DCM/MeOH (10:1). Compound 1: pyridin-4-ylmethyl-7-(diethylamino)-2-oxo-2H-chromene-3-carboxylate was obtained as a yellow powder, yield: 63%.
¹H NMR (400 MHz, Methanol-d₄) δ 8.68 - 8.59 (m, 3H), 7.75 - 7.69 (m, 2H), 7.55 (d, J = 9.0 Hz, 1H), 6.81 (dd, J = 9.1, 2.5 Hz, 1H), 6.55 (d, J = 2.4 Hz, 1H), 5.46 (s, 2H), 3.54 (q, J = 7.1 Hz, 4H), 1.24 (t, J = 7.1 Hz, 6H);
¹³C NMR (151 MHz, Methanol-d₄) δ 163.68, 159.14, 158.66, 153.86, 150.27, 149.19, 147.51, 131.66, 122.55, 122.52, 110.18, 107.69, 106.05, 95.89, 63.96, 44.67, 11.28, -1.44.

### Example 2: Synthesis of Compound 2

Based on a synthesis method similar to Example 1, Compound 2: pyridin-3-ylmethyl-7-(diethylamino)-2-oxo-2H-chromene-3-carboxylate was obtained as a yellow powder, yield: 75%.
¹H NMR (400 MHz, DMSO-d₆) δ 8.99 (s, 1H), 8.84 (d, J = 5.5 Hz, 1H), 8.67 (s, 1H), 8.54 - 8.51 (m, 1H), 7.99 - 7.96 (m, 1H), 7.66 (d, J = 9.1 Hz, 1H), 6.80 (dd, J = 9.1, 2.4 Hz, 1H), 6.56 (d, J = 2.4 Hz, 1H), 5.46 (s, 2H), 3.48 (q, J = 7.0 Hz, 4H), 1.14 (t, J = 7.0 Hz, 6H);
¹³C NMR (151 MHz, DMSO-d6) δ 163.63, 158.75, 157.60, 153.62, 150.56, 149.95, 143.71, 143.17, 143.00, 135.92, 132.49, 132.34, 126.68, 110.54, 110.47, 107.59, 106.65, 96.39, 96.31, 79.68, 63.00, 44.90, 12.82, 12.80

### Example 3: Synthesis of Compound 3

Based on a synthesis method similar to Example 1, Compound 3: pyridin-2-ylmethyl-7-(diethylamino)-2-oxo-2H-chromene-3-carboxylate was obtained as a yellow powder, yield: 61%.
¹H NMR (400 MHz, Methanol-d₄) δ 8.63 (s, 1H), 8.54 - 8.52 (m, 1H), 7.90 - 7.86 (m, 1H), 7.65 (d, J = 7.8 Hz, 1H), 7.51 (d, J = 9.0 Hz, 1H), 7.39 - 7.36 (m, 1H), 6.78 (dd, J = 9.1, 2.5 Hz, 1H), 6.52 (d, J = 2.4 Hz, 1H), 5.38 (s, 2H), 3.52 (q, J = 7.1 Hz, 4H), 1.23 (t, J = 7.1 Hz, 6H);
¹³C NMR (151 MHz, Methanol-d4) δ 164.59, 160.88, 159.27, 158.42, 153.62, 149.83, 147.91, 137.48, 131.43, 122.32, 120.74, 110.06, 107.57, 106.69, 95.86, 64.06, 11.29.

### Example 4: Synthesis of Compound 4

Based on a synthesis method similar to Example 1, Compound 4: quinolin-4-ylmethyl-7-(diethylamino)-2-oxo-2H-chromene-3-carboxylate was obtained as a yellow powder, yield: 56%.
¹H NMR (400 MHz, DMSO-d₆) δ 8.93 (d, J = 4.4 Hz, 1H), 8.68 (s, 1H), 8.15 (d, J = 8.4 Hz, 1H), 8.09 (d, J = 8.4 Hz, 1H), 7.82 (t, J = 7.7 Hz, 1H), 7.74 - 7.64 (m, 3H), 6.79 (dd, J = 9.0, 2.4 Hz, 1H), 6.57 (d, J = 2.3 Hz, 1H), 5.84 (s, 2H), 3.49 (q, J = 7.1 Hz, 4H), 1.14 (t, J = 7.0 Hz, 6H);
¹³C NMR (151 MHz, DMSO-d6) δ 163.71, 158.77, 157.59, 157.09, 153.59, 150.93, 150.47, 148.01, 142.15, 132.53, 130.11, 130.01, 127.44, 125.76, 124.20, 119.74, 110.42, 107.63, 106.89, 96.32, 63.00, 47.97, 44.89, 33.83, 25.80, 24.94, 12.82.

### Example 5: Synthesis of Compound 5

Based on a synthesis method similar to Example 1, Compound 5: 7-(diethylamino)-2-oxo-2H-chromene-3-carboxylic acid quinolin-3-ylmethyl ester was obtained as a yellow powder, yield: 58%.
¹H NMR (400 MHz, Chloroform-d) δ 9.06 (d, J = 2.1 Hz, 1H), 8.54 (s, 1H), 8.46 (s, 1H), 8.32 (d, J = 8.6 Hz, 1H), 7.95 (d, J = 8.2 Hz, 1H), 7.82 (t, J = 7.7 Hz, 1H), 7.66 (t, J = 7.6 Hz, 1H), 7.36 (d, J = 9.0 Hz, 1H), 6.61 (dd, J = 9.1, 2.4 Hz, 1H), 6.46 (d, J = 2.4 Hz, 1H), 5.58 (s, 2H), 3.45 (q, J = 7.1 Hz, 4H), 1.24 (t, J = 7.1 Hz, 6H)

### Example 6: Synthesis of Compound 6

Based on a synthesis method similar to Example 1, Compound 6: 7-(diethylamino)-2-oxo-2H-chromene-3-carboxylic acid quinolin-2-ylmethyl ester was obtained as a yellow powder, yield: 54%.
¹H NMR (400 MHz, Chloroform-d) δ 8.55 (s, 1H), 8.28 (d, J = 8.5 Hz, 1H), 8.18 (d, J = 8.5 Hz, 1H), 7.89 - 7.72 (m, 3H), 7.58 (t, J = 7.5 Hz, 1H), 7.37 (d, J = 9.0 Hz, 1H), 6.61 (dd, J = 9.0, 2.5 Hz, 1H), 6.48 (d, J = 2.4 Hz, 1H), 5.70 (s, 2H), 3.46 (q, J = 7.1 Hz, 4H), 1.24 (t, J = 7.1 Hz, 6H).

### Example 7: Synthesis of Compound 7

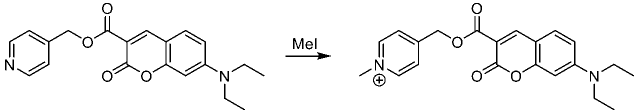

Compound 1 (100 mg, 0.284 mmol, 1.0 eq.) was dissolved in a 1:1 methanol-iodomethane mixed solution (5 mL). The reaction mixture was stirred at 80 °C for 2 hours, evaporated under reduced pressure, and the crude product was purified by high-performance liquid chromatography eluting with water/MeCN (70:30). Compound 7: 4-((7-(diethylamino)-2-oxo-2H-chromene-3-carbonyl)oxy)methyl-1-methylpyridin-1-ium was finally obtained as a yellow powder, yield: 90%.
¹H NMR (400 MHz, DMSO-d6) δ 9.03 (d, J = 6.4 Hz, 2H), 8.75 (s, 1H), 8.20 (d, J = 6.2 Hz, 2H), 7.69 (d, J = 9.0 Hz, 1H), 6.83 (dd, J = 9.0, 2.4 Hz, 1H), 6.58 (d, J = 2.3 Hz, 1H), 5.62 (s, 2H), 4.36 (s, 3H), 3.51 (q, J = 7.0 Hz, 4H), 1.15 (t, J = 7.0 Hz, 6H).

### Example 8: Synthesis of Compound 8

Based on a synthesis method similar to Example 7, Compound 8: 3-((7-(diethylamino)-2-oxo-2H-chromene-3-carbonyl)oxy)methyl-1-methylpyridin-1-ium was obtained as a yellow powder, yield: 93%.
¹H NMR (400 MHz, Methanol-d₄) δ9.15 (s, 1H), 8.88 (d, J = 6.1 Hz, 1H), 8.70 (d, J = 8.2 Hz, 1H), 8.66 (s, 1H), 8.54 (s, 1H), 8.15-8.07 (m, 1H), 7. 54 (d, J = 9.0 Hz, 1H), 6.82 (dd, J = 9.1, 2.5 Hz, 1H), 6.55 (d, J = 2.4 Hz, 1H), 5.54 (s, 2H), 4.46 (s, 3H), 3.55 (q, J = 7.1 Hz, 4H), 1.24 (t, J = 7.1 Hz, 6H).
¹³C NMR (151MHz, DMSO-d₆) δ 163.44, 158.79, 157.57, 153.71, 150.70, 145.44, 144.66, 144.24, 137.55, 132.51, 127.96, 110.55, 107.57, 106.38, 96.32, 62.35, 48.64, 44.91, 44.44, 12.84, 12.82.

### Example 9: Synthesis of Compound 9

Based on a synthesis method similar to Example 7, Compound 9: (2-((7-(diethylamino)-2-oxo-2H-chromene-3-carbonyl)oxy)methyl-1-methylpyridin-1-ium) was obtained as a yellow powder, yield: 89%.

### Example 10: Synthesis of Compound 10

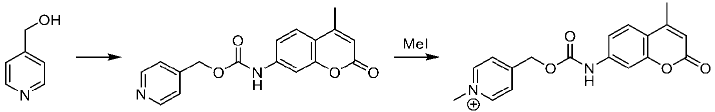

7-(dimethylamino)-4-methylcoumarin (1.75 g, 10 mmol, 1.0 eq.) and triphosgene (1.48 g, 5 mmol, 0.5 eq.) were dissolved in DCM (35 mL, anhydrous). DIPEA (3.87 g, 30 mmol, 3.0 eq.) was added under N₂ atmosphere. The reaction mixture was stirred for 30 minutes and became transparent. This mixture was used for the next step without purification. Pyridin-4-ylmethanol (10a) (1.20 g, 11 mmol, 1.1 eq.) was added. The reaction mixture was stirred at room temperature for 4 hours, then evaporated under reduced pressure, and the crude product was purified by flash chromatography eluting with DCM/MeOH (10:1) to give Compound 10b as a pink powder, yield: 72%.

Compound 10b (100 mg, 0.322 mmol, 1.0 eq.) was dissolved in a 1:1 methanol-iodoethane mixed solution (5 mL). The reaction mixture was stirred at 65 °C for 2 hours, evaporated under reduced pressure, and the crude product was purified by high-performance liquid chromatography eluting with water/MeCN (70:30) to give Compound 10 as a pink powder, yield: 83%.

### Example 11: Synthesis of Compound 11 (NAPC-MeRho)

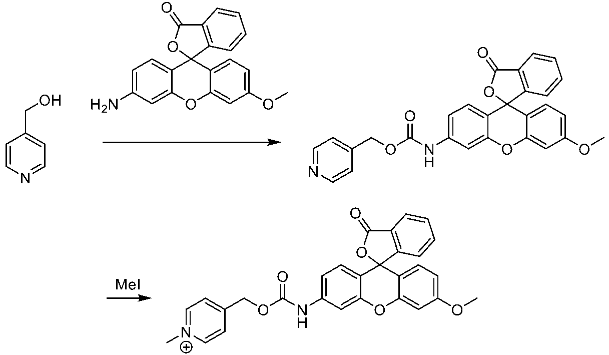

Synthetic route for **NAPC-MeRho: MeRho (17)** (1.75 g, 10 mmol, 1.0 eq.) and triphosgene (1.48 g, 5 mmol, 0.5 eq.) were dissolved in DCM (35 mL, anhydrous). DIPEA (3.87 g, 30 mmol, 3.0 eq.) was added under N₂ atmosphere. The reaction mixture was stirred for 30 minutes and became transparent. This mixture was used for the next step without purification. 14 (1.20 g, 11 mmol, 1.1 eq.). The reaction mixture was stirred at room temperature for 4 hours, then evaporated under reduced pressure, and the crude product was purified by column chromatography eluting with DCM/MeOH (10:1). **15** was obtained as an orange powder. **18** (100 mg, 0.322 mmol, 1.0 eq.) was dissolved in a 1:1 methanol-iodoethane mixed solution (5 mL). The reaction mixture was stirred at 65 °C for 2 hours, evaporated under reduced pressure, and the crude product was purified by high-performance liquid chromatography eluting with water/MeCN (70:30). **NAPC-MeRho** was obtained as an orange powder (yield: 69%)

### Example 12: Synthesis of Drug Conjugate (ADC)

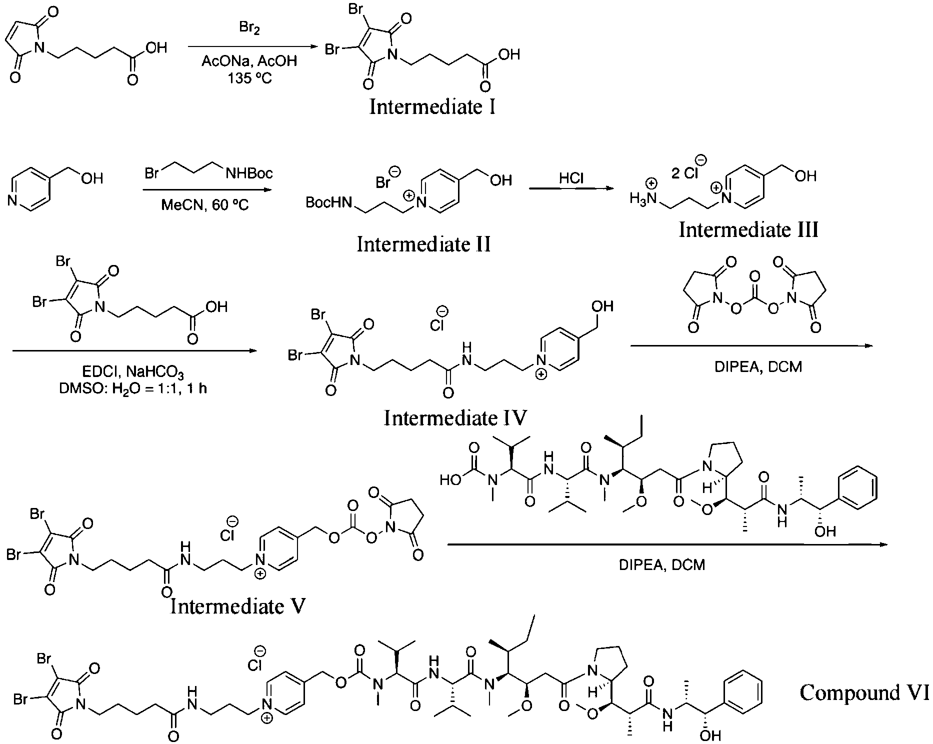

(1) Synthesis of Intermediate I: Dissolve 6-maleimidocaproic acid (840 mg, 4.0 mmol, 1.0 eq.) and sodium acetate (720 mg, 8.8 mmol, 2.2 eq.) in acetic acid (8 mL) in a tube at 0 °C; and a mixture of bromine (450 µL, 8.8 mmol, 2.2 eq.) was added and the tube was sealed. The mixture was stirred at 135 °C for 4 hours; after cooling to 0 °C, 10 mL water was added to the mixture; the solution was extracted with ethyl acetate (3 × 15 mL); combined organic phases were washed with sodium thiosulfate (2 × 15 mL); the reaction mixture was dried over magnesium sulfate; the reaction mixture was concentrated under reduced pressure; residual acetic acid was co-evaporated with toluene under reduced pressure; the crude residue was purified by flash chromatography to obtain the Intermediate I.
(2) Synthesis of Intermediate II: Pyridin-4-ylmethanol (2 g, 18.32 mmol, 1.0 eq.) and tert-butyl (3-bromopropyl)carbamate (5.24 g, 22.00 mmol, 1.2 eq.) were dissolved in 10 mL MeCN; the reaction was monitored by thin-layer chromatography (TLC); after complete consumption of the alcohol, solid residue was discarded, and the liquid residue was purified by flash chromatography to obtain the Intermediate II.
(3) Synthesis of Intermediate III: Concentrated HCl (5 mL) was added to the Intermediate II (1066 mg, 3.1 mmol) and stirred for 5 minutes; subsequently, the reaction mixture was concentrated under reduced pressure and lyophilized.
(4) Synthesis of Intermediate IV: Intermediate III (800 mg, 2.2 mmol, 1.0 eq.), NaHCO₃ (370 mg, 4.4 mmol, 2.0 eq.) and Intermediate I (1000 mg, 2.6 mmol, 1.2 eq.) were dissolved in 20 mL H₂O:DMSO=1:1; the reaction was monitored by thin-layer chromatography (TLC). After completion, water and ethyl acetate were added to the reaction mixture. The organic layer was dried over anhydrous magnesium sulfate and concentrated under reduced pressure. Purification was performed using a reverse-phase C-18 column.
(5) Synthesis of Intermediate V: Intermediate IV (300 mg, 0.56 mmol, 1.0 eq.), DCM (20 mL) and DIPEA (0.30 mL, 1.68 mmol, 3.0 eq.) were placed in a round-bottom flask equipped with a stir bar; DSC (430 mg, 1.68 mmol, 3.0 eq.) was added to the reaction mixture, stirred at room temperature for 16 hours, the solvent was evaporated, and the crude material was purified by reverse-phase C-18 column chromatography to obtain the Intermediate V.
(6) Synthesis of Compound VI: The Intermediate V (30 mg, 0.044 mmol, 1.0 eq.), DCM (2 mL) and DIPEA (7.7 µL, 0.044 mmol, 1.0 eq.) and MMAE (57 mg, 0.044 mmol, 1.0 eq.) were placed in a round-bottom flask equipped with a stir bar, stirred at room temperature for 16 hours, the solvent was evaporated, and the crude material was purified by reverse-phase C-18 column chromatography to obtain the product VI.
(7) The antibody (150 kDa) was dissolved in ADC buffer (pH 7.4) to obtain a protein solution of 10 mg/mL (66 µM) and reduced with 4.8 eq. of TCEP at 37 °C for 1 hour. Then 5 eq. of Compound VI was added, and the reaction was maintained at 37 °C for 1 hour. The maleimide is quantitatively inserted into disulfide bonds to obtain antibody-conjugated drugs.

### Example 13: Radiation Experiments

The following procedures were used to irradiate compounds:

### Deoxygenation Step

Pretreated solution (approx. 1-2 mL) was sealed in glass vials with rubber stoppers and metal caps. All tubular radiation experiments were pretreated using the freeze-thaw method to remove dissolved oxygen from the solution. The solution was frozen, then vacuum was applied for several minutes until thawed to room temperature.

### Irradiation Step

X-ray irradiation was performed using an X-ray generator (RS2000 Pro 225, 225 kV, 17.7 mA; Rad Source Technologies, Inc).

Tubes were irradiated with X-rays, with a total dose of 0-60 Gy, at a rate of 2 Gy/min. Cells were irradiated with X-rays at different doses at a rate of 2 Gy/min.

Local tumor irradiation dose was 4 Gy, dose rate 2 Gy/min, with the rest of the body shielded by 5 mm thick lead.

Gamma-ray irradiation was provided by a ⁶⁰Co source.

The following procedures were used for analysis:

### Standard Solutions and Calibration Curves

1. Standard preparation and calibration curves. The standard (coumarin, MeRho or MMAE) was dissolved in DMSO solution to make a 10 mM stock solution. The stock was diluted with deionized water to obtain solutions of suitable concentration gradients. These standard solutions were analyzed by UPLC-MS to generate calibration curves for the respective standard compounds. The peak areas of the target compounds were within the linear range of the curves. The relative standard deviation for duplicate injections of each standard solution was less than 5.0%.
2. Analysis and calculation. After different treatments, the reaction mixtures were analyzed by ultra-performance liquid chromatography-mass spectrometry. The amount of cargo was calculated as equivalents of their respective external standard.

### Detection of Compound Release

### 1) Radiation

As a representative example, the detailed procedure for X-ray-induced reduction of Compound 1 is described below. A stock solution of Compound 1 (10 mM in DMSO) was diluted in PB (0.02 M, pH 7.4) to a final concentration of 10 µM. Solutions were deoxygenated using the aforementioned deoxygenation step prior to X-ray irradiation from 0 to 60 Gy. Reaction mixtures were analyzed using UPLC and a fluorescence spectrometer, and amount of product release was quantified via standard curves.

### 2) Using Reducing Solutions

20 µM NAPC-MeRho was mixed with an equal volume of 2 mM sodium ascorbate solution, incubated and detected at different time points using a fluorescence spectrometer and UPLC-MS. Stability test results in cysteine and γ-glutathione solutions were the same.

### UPLC-MS Methods

The elution program for Compounds 1-9 and NAPC-MeRho was Method a: H₂O + 5% ACN (0 min)-95% H₂O + 5% ACN (0.2 min)-5% H₂O + 95% ACN (2.8 min)-5% H₂O + 95% ACN (2.9 min)-95% H₂O + 5% ACN (3.0 min)-95% H₂O + 5% ACN (4.0 min). Injection volume: 10 µL.

Method B was used for elution of Compound 10, mixtures of Compound 7 with other RPGs, and mixtures of Compound 10 with other rpg: 95% H₂O + 5% ACN (0 min)-95% H₂O + 5% ACN (0.2 min)-70% H₂O + 30% ACN (3.0 min)-70% H₂O + 30% ACN (3.6 min)-95% H₂O + 5% ACN (3.7 min)-95% H₂O + 5% ACN (5.0 min). Injection volume: 10 µL.

### Example 13a: Product Analysis Proving C-O Bond Cleavage After Single Electron Reduction of 4-Pyridinium Ester

In Compound 1, the reporter molecule 7-(diethylamino)-2-oxo-2H-chromene-3-carboxylic acid (7-Deacca) is blocked by the 4-pyridyl group.

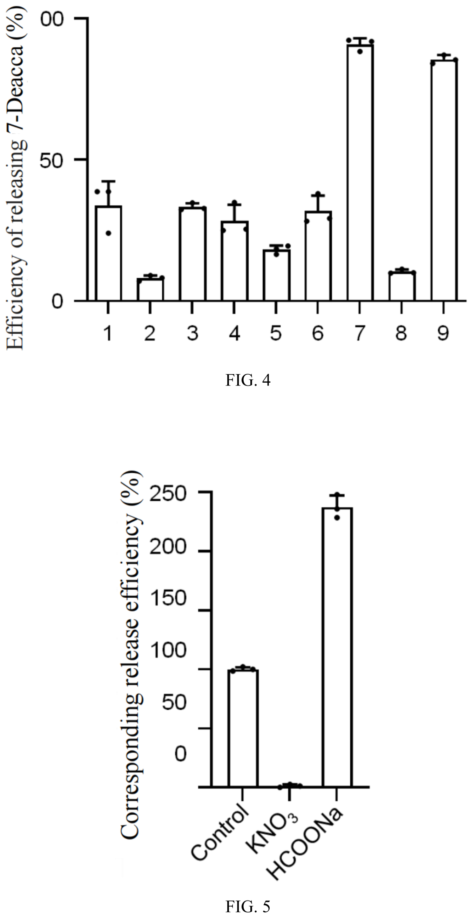

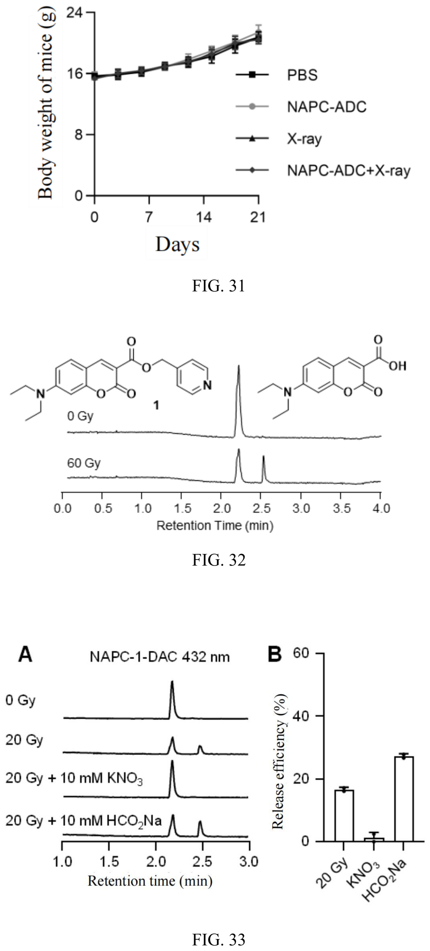

10 mM KNO₃ was added to the radiation system of Compound 1 (10 µM, in PB, pH 7.4)
Reactivity test (10 µM, 0.1% DMSO): Determination of 7-Deacca release in PB (20 mM, pH 7.4) after deoxygenation and 60 Gy X-ray irradiation. Encouragingly, UPLC-MS detected coumarin release (FIG. 32), with a release efficiency of 33.5 ± 9.1% (FIG. 4), resulting in 55.8 nM/Gy.

Relative to the control group, the release efficiency in the KNO₃ solution of this example was significantly reduced (FIG. 5 and FIG. 33), indicating that e⁻_{aq} plays a key role in the reaction when KNO₃ quenches e⁻_{aq}.

### Example 13b: Radiation Reduction Efficiency of RPGs

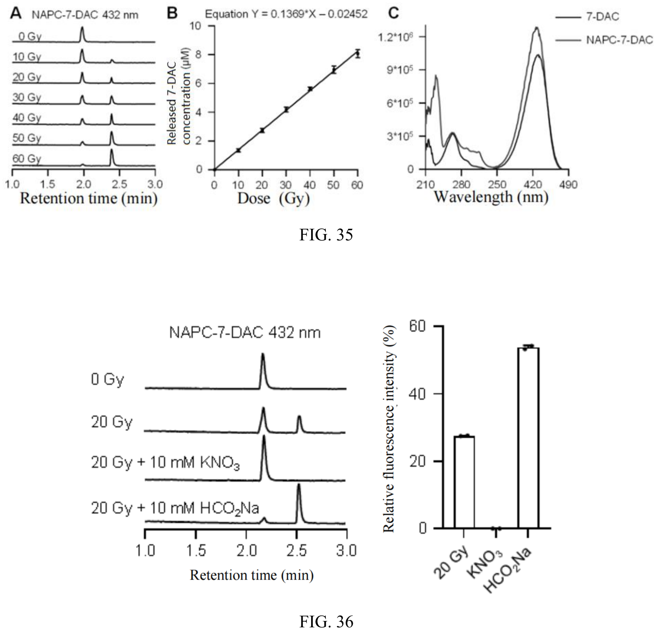

7-Deacca was designed to be protected by a series of molecules Compounds 2-9 with pyridyl, quinolinyl, and N-methylpyridyl groups. Stock solutions of Compounds 2-9 (10 mM in DMSO) were diluted in PB (10 µM, 0.1% DMSO), then irradiated with 60 Gy X-rays, and analyzed by UPLC-MS to determine the yield of free 7-Deacca (FIG. 35).

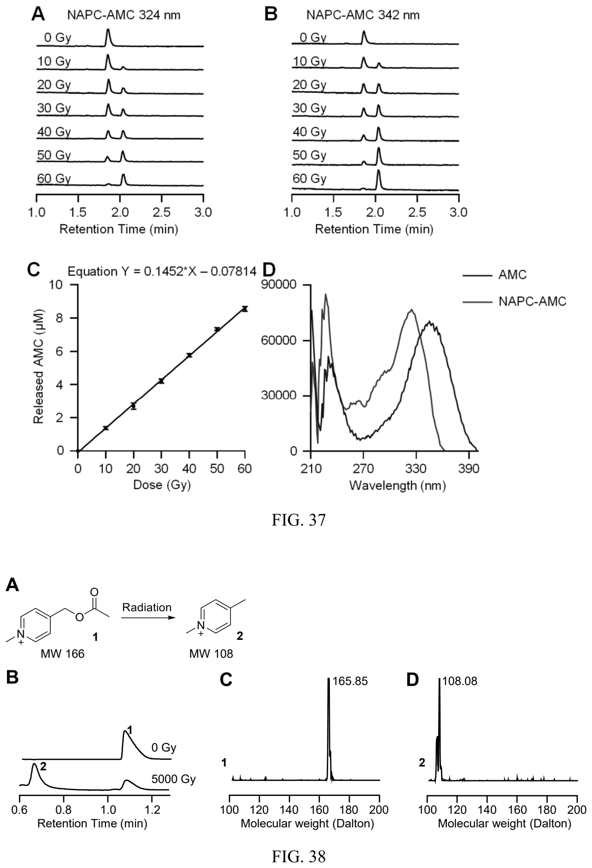

The inventors found that the amount of 7-Deacca released from Compounds 1-9 is determined by the substitution position and electron density on the pyridyl moiety. More specifically, ortho- and para-substituted esters (Compounds 1, 3, 4, 6, 7, and 9) are more reactive to radiation reduction than meta-substituted ones (Compounds 2, 5, and 8). The NAP esters of Compounds 7 and 9 particularly have high release efficiencies. When Compound 7 was irradiated with an X-ray dose gradient from 0 to 60 Gy, the release of the reporter molecule showed dose dependency as detected by UPLC (FIG. 37), with a G-value of 140 nM/Gy.

### Example 13c: Compound 7 is Reduced via Radiation-Mediated Hydrated Electron Reduction Mechanism

10 mM HCOONa or KNO₃ was added to the radiation system of Compound 7 (10 µM, in PB, pH 7.4). Compound 7 showed the same result as Compound 1 in the KNO₃ solution, with significantly reduced release efficiency (FIG. 5 and FIG. 36), while the addition of HCOONa approximately doubled the reduction yield of Compound 7. It can be explained that radicalized HCOONa in water reacts with ·OH and hydrogen radicals (·H) to generate carboxylate radicals (·CO²⁻), which is another reducing agent with a standard electrode potential of -1.90 V, which may also be a possible explanation for the difference in its radiation reduction efficiency. Compared to the quinolinyl moiety with higher electron density (lower reduction potential), the positive charge on the pyridyl moiety with lower electron density (higher reduction potential) has higher affinity for the negatively charged e⁻_{aq}, resulting in the highest radiation reduction efficiency among them for the pyridyl moiety.

### Example 13d: Adaptability of RPG

Besides carboxyl groups, the amide group is another important moiety in many bioactive molecules due to its ability to form hydrogen bonds with residues in the target binding pocket. The inventors demonstrated that the RPG is adaptable using an N-alkyl-4-pyridinium carbamate (NAPC) system (where the carbamate carbon is attached to the reporter molecule) to release functional molecules in their natural and unmodified active form.

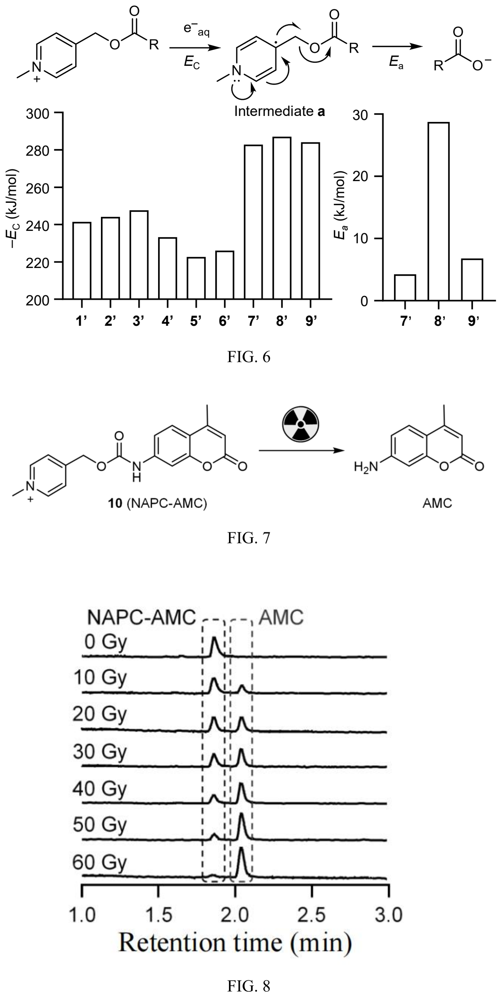

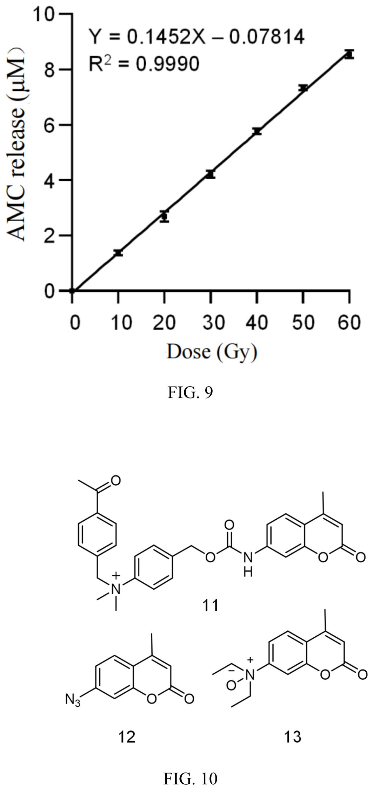

The inventors synthesized Compound 10 (NAPC-AMC) containing NAP-protected 7-amino-4-methylcoumarin (AMC). This compound releases AMC via C-O bond cleavage and decarboxylation upon receiving radiation (FIG. 7), which is in a dose-dependent manner (FIG. 8). The amount of AMC release is promoietyal to the radiation dose (FIG. 9), indicating that the radiation reduction reaction occurs stoichiometrically (G = 145 nM/Gy).

Recently, radiotherapy-induced controlled release systems have flourished, such as ortho-carbamoyl methylene quaternary ammonium (Compound 11), azide (Compound 12), and N-oxide (Compound 13) systems (FIG. 10). Although they are all competent e⁻_{aq} acceptors, their ability to react with e⁻_{aq} under the same conditions is different. Biological systems are complex and unpredictable, with numerous redox processes present, and radiation reduction reactions are highly likely to be interfered with by oxidizing species. A higher cross-section for the radiation reduction reaction means greater competence for e⁻_{aq}, thereby resulting in higher release efficiency in biological environments.

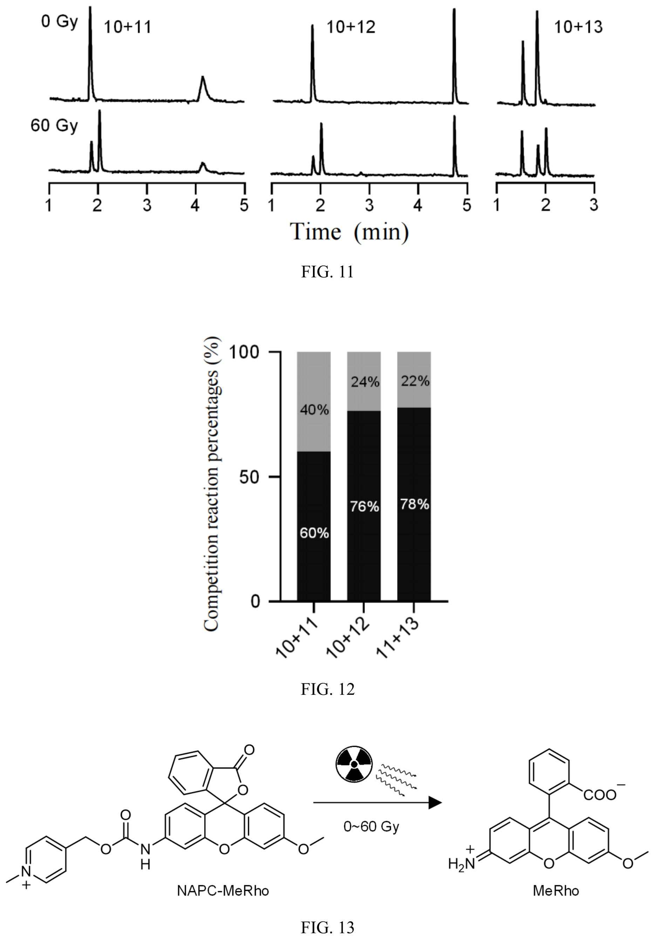

The inventors mixed the reported RPGs with Compound 10, providing solutions of [10+11], [10+12], [10+13], respectively (each component: 10 µM in PB, pH 7.4). The solutions were deoxygenated and irradiated with 60 Gy X-rays, and their release efficiencies were analyzed by UPLC-MS (FIG. 11) and summarized in FIG. 12. The lower part of the bars and the upper part of the bars represent the activation ratio of Compound 10 and each competing component in the irradiation system, respectively. Competition inhibition experiments show that the NMP group is a more reactive single-electron reduction substrate than previously reported structures, producing 20-40% more under the same concentration.

### Example 14: POC in Cells and Tumors

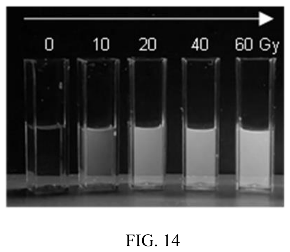

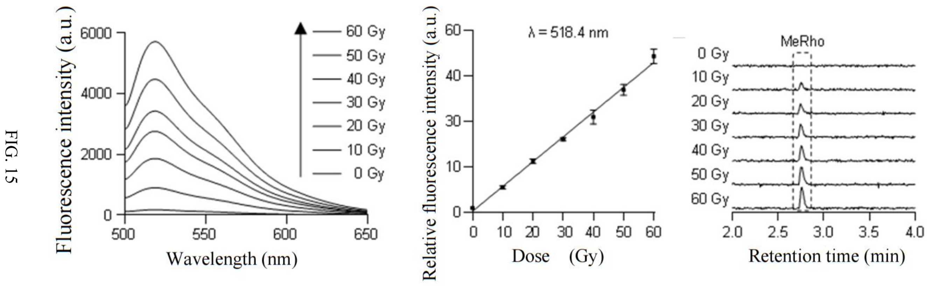

The inventors performed proof-of-concept for a radiation-responsive fluorescent probe NAPC-MeRho (FIG. 13), designed for quantitative analysis of radiation-induced release. The NAPC linker selectively responds to e⁻_{aq} generated by X-rays, where C-O bond cleavage of the carbamate occurs, followed by decarboxylation to release fluorescent MeRho (FIG. 13). Under physiological conditions, irradiating 10 µM NAPC-MeRho with a gradient dose from 0 to 60 Gy caused fluorescence intensity to increase in a linear manner (FIG. 14 and FIG. 15), and UPLC spectra determined a G-value for MeRho release of 140 nM/Gy (FIG. 15). To test whether the radiation reduction reaction can occur in living cells without interference, stability to biological reducing agents and cytotoxicity to different cell lines were evaluated. NAPC-MeRho (10 µM) was highly stable when treated with 100 eq. VcNa at pH 7.4, and cell viability with 10 µM NAPC-MeRho was greater than 95%.

Typically, cells were seeded at a density of 2×10⁵ cells/mL in 35-mm confocal dishes and cultured under hypoxic conditions in an incubator containing 5% CO₂ at 37 °C for 24 hours. A stock solution of NAPC-MeRho (10 mM) was prepared in DMSO. Attached cells were washed twice with PBS before adding the fluorescence probe at a final concentration of 10 µM in 1 mL deoxygenated HBSS. After hypoxic incubation at 37 °C for 30 min, they were subjected to X-ray irradiation for different times (0, 4, 8, 16 Gy), and fluorescence images were taken with a laser scanning confocal microscope (Nikon, Japan). Excitation wavelength was 488 nm, fluorescence emission window was 500-530 nm. For imaging quantification, all cells on the image were set as regions of interest, and fluorescence intensity was measured using Nikon imaging software (version 4.0).

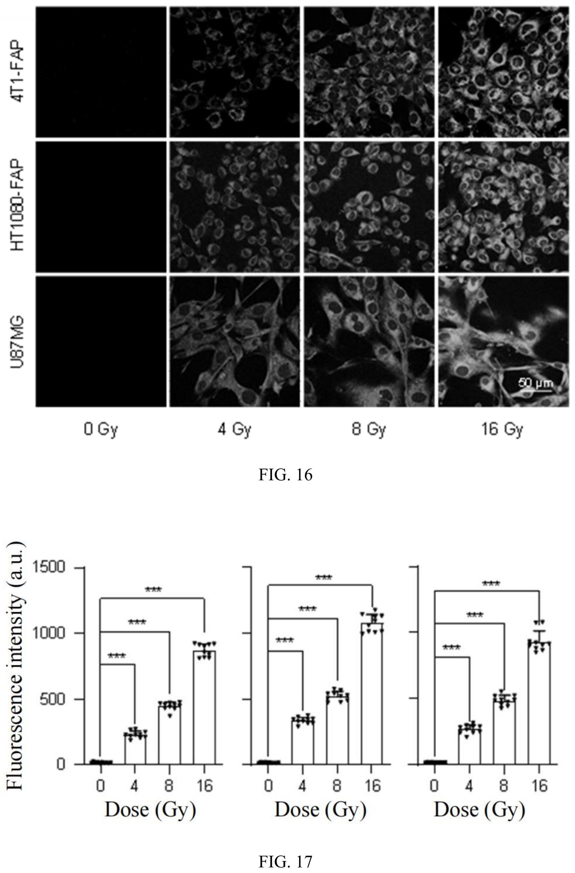

4T1-FAP, HT1080-FAP, and U87MG cell lines incubated with 10 µM NAPC-MeRho under hypoxic conditions for 30 minutes showed no spontaneous release of MeRho (FIG. 16, left lane). Subsequent irradiation with 4, 8, and 16 Gy presented the appearance of green fluorescence from MeRho release (FIG. 16, right lanes), with fluorescence intensity positively correlated with radiation dose (FIG. 17). Under 4 Gy, 8 Gy, and 16 Gy radiation, fluorescence intensity in 4T1-FAP cells increased by 14.4-, 27.9-, and 54.5-fold, respectively (FIG. 17, left). Similar experimental results were obtained in HT1080-FAP cells (FIG. 17, middle) and U87MG cells (FIG. 17, right), indicating that NAPC-MeRho is stable and ready for radiation reduction reactions in mammalian cells.

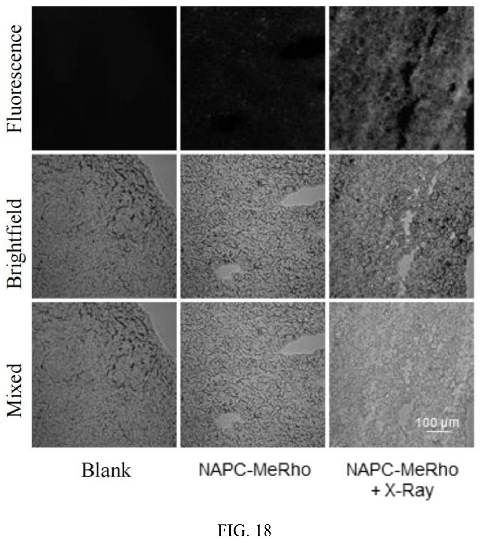

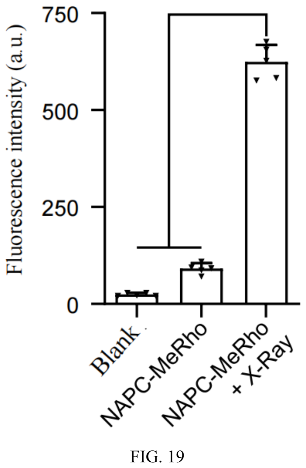

Then, when examining the reduction of NAPC-MeRho in tumor-bearing mice, 20 µL of 100 µM NAPC-MeRho in PBS was locally injected into 4T1-FAP tumors, and X-ray therapy of 4 Gy was administered 30 minutes post-injection. Due to limited green light penetration, tumors were harvested and quickly frozen, and fluorescence intensity was quantified via confocal imaging. When treated with NAPC-MeRho only, limited fluorescence signal was detected in tumor sections (FIG. 18, middle lane), which significantly increased when additional radiation was delivered (FIG. 18, bottom lane). Furthermore, the MeRho signal was statistically greater than the non-irradiated group (FIG. 19), providing conclusive evidence that radiation reduction reactions are feasible *in vivo.* The inventors successfully demonstrated that radiation activation of fluorescence probes with NAPC structure is feasible both in cells and tumors, with suitable stability and reactivity in complex biological environments.

Cell viability was assessed using CCK-8 assay. Each experiment was performed in triplicate. To detect cytotoxicity of MMAE and NAPC-ADC, 4T1-FAP cells were seeded at 5×10⁴ /mL in 96-well plates in 200 µL rmi-1640 medium containing 10% FBS and 1% penicillin/streptomycin, incubated at 37 °C in a 5% CO₂ incubator for 24 hours, then incubated with different concentrations of MMAE and NAPC-ADC for 72 hour, and blank medium containing CCK-8 at a final concentration of 0.5 mg/mL was added. Incubated at 37 °C, 5% CO₂ for 2 hours, absorbance was measured at 450 nm. The absorbance of treated cells was compared to that of the control group, whose viability was set at 100%.

To evaluate cytotoxicity of 4T1-FAP cells under different treatment conditions, 4T1-FAP cells were seeded at 5×10⁴ /mL in 96-well plates in 200 µL rmi-1640 medium containing 10% FBS and 1% penicillin/streptomycin, cultured at 37 °C in a 5% CO₂ incubator for 12 hours, then incubated with 100 nM NAPC-ADC under hypoxic conditions for 24 hours. Cells were irradiated with X-ray at 4 Gy, and after incubation for 48 hours, blank medium containing CCK-8 at a final concentration of 0.5 mg/mL was added. Incubated at 37 °C in a 5% CO2 incubator for 2 hours, absorbance was measured at 450 nm. The absorbance of treated cells was compared to that of the control group, whose viability was set at 100%.

4T1-FAP and HT1080-FAP cells were seeded in 8-well plates (5×10⁴ cells/well) and adhered overnight under hypoxic conditions. Then cells were treated with NAPC-ADC (100 nM), X-ray (4 Gy), or combination for 12 hours, then fixed in cold methanol (4 °C) for 30 min, followed by staining with fitc-labeled anti-α-tubulin (Abbkine) at room temperature (1:100 v:v; λex = 488; λem = 570 nm). Nuclei were stained with DAPI. All samples were imaged using a Nikon confocal laser scanning microscope.

### Example 15: ADC Activation for In Vivo Anticancer Activity

The following steps were used for measuring MMAE concentration after *in vivo* ADC activation:
Six-week-old female Balb/c mice were ordered from Beijing Vital River Laboratory Animal Technology Co., Ltd., housed under specific pathogen-free conditions with free access to standard food and water. About 2×10⁶ 4T1-FAP cells suspended in 100 µL PBS were subcutaneously implanted into the right shoulder of mice for PET imaging, fluorescence imaging, and treatment. Tumor volume was calculated as: volume = 1/2 × length × width². When tumor volume reached about 100-200 mm³ (10-15 days post-implantation), fluorescence imaging and PET imaging were performed. When tumor volume reached 40-100 mm³ (7 days post-implantation) was the optimal time for treatment experiments. Mice were euthanized if tumor volume exceeded 1500 mm³, and treatment duration was 21 days.

Four groups of mice bearing BGC823 xenografts were injected with NC-ADC or oxaliPt(IV)-ADC (5 mg/kg). 48 hours after ADC administration, X-ray irradiation was given. Then mice were euthanized, and tumor samples were collected from each group and weighed. Tumor (0.1-0.2 g) was transferred to a glass tube with 1 mL methanol. Tissue was homogenized, centrifuged (13000 rpm, 5 min) to remove debris. Methanol was evaporated, residue was reconstituted in water containing 10% acetonitrile (500 µL) and filtered through a 0.22 µm filter.

All samples were analyzed by LC-QTOF-MS, and free MMAE content was quantified according to standard curves. The detection limit for MMAE by this method was 5 nM.

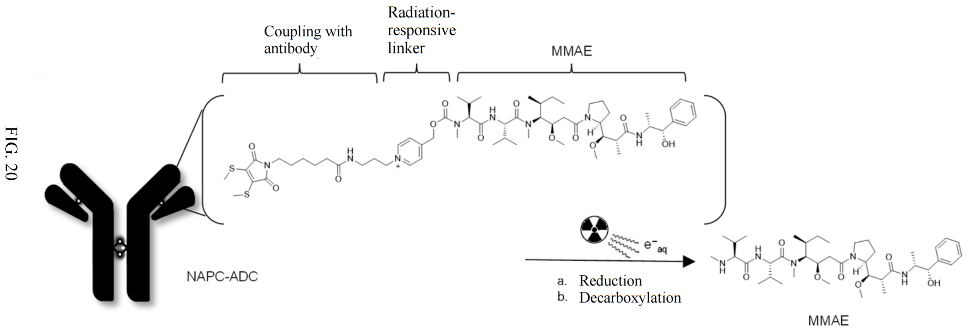

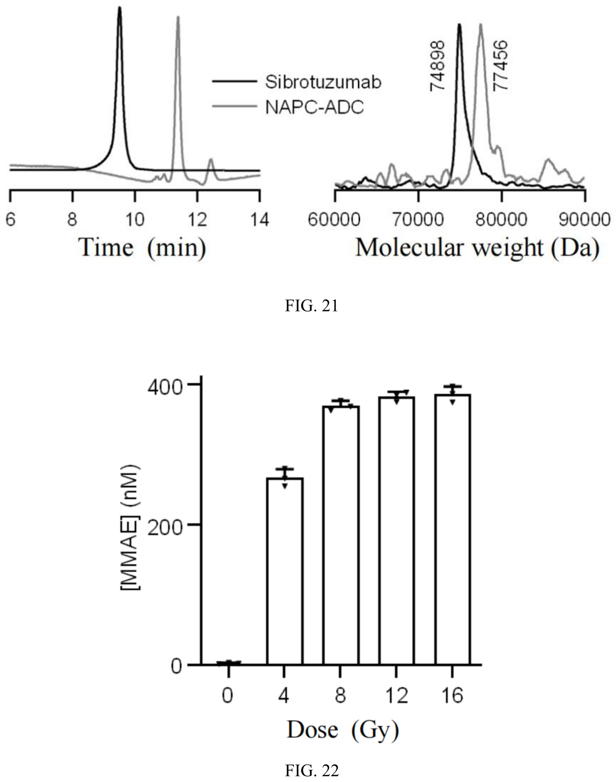

Antibody drug conjugates (ADCs) are a perfect example of prodrugs with extremely high tumor selectivity in clinical development, but their release is uncontrolled and inefficient, thus requiring third-generation ADCs with excellent stability and reactivity. Radiotherapy-induced reduction reactions can be applied to ADCs, providing a new platform to address this problem and improve drug release efficiency in tumors. Based on model reactions, MMAE was conjugated to a humanized monoclonal antibody derived from Sibutramine via conventional chemical operations using a pyridinium linker (FIG. 20). This antibody has been developed for fibroblast activation protein (FAP)-targeted radionuclide therapy, with a mass of 149.8 kDa and a binding affinity of 0.608 nM. In this case, the N-alkyl-4-pyridinium carbamate (NAPC) linker is selectively reduced by e⁻_{aq} generated from radiation to cleave the C-O bond, followed by decarboxylation and release of the highly toxic free MMAE (FIG. 20). By hydrophobic interaction chromatography high-performance liquid chromatography (HIC HPLC) analysis, the Cetuximab-(N-alkyl-4-pyridinium carboxylate)-MMAE conjugate (NAPC-ADC) is a homogeneous ADC, as evidenced by a single main peak (FIG. 21). By MALDI-TOF analysis, the drug-to-antibody ratio (DAR) of the modified ADC is 4 (FIG. 21), indicating the main peak comprises 4 payloads per antibody (FIG. 21), which is optimal for *in vivo* tumor-specific drug delivery.

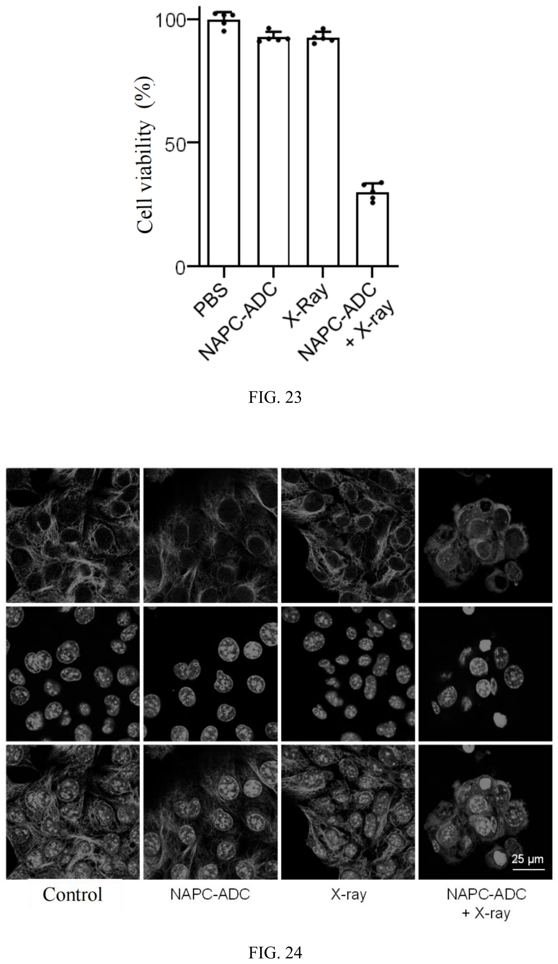

It was demonstrated that 4 Gy irradiation can release over 220 nM MMAE from 100 nM ADC in PB (pH 7.4) (FIG. 22), which is sufficient for tumor treatment, as the measured IC50 for free MMAE is below 1.694 nM. The inventors also observed that ADC (100 nM) was activated in a radiation dose-dependent manner, reaching nearly 100% conversion at 8 Gy radiation. To test whether radiation-induced release would achieve MMAE release from ADC, cell viability assays with NAPC-ADC + X-ray were performed using 4T1-FAP and HT1080-FAP cell lines (FIG. 23). Cells were treated with X-ray alone, NAPC-ADC alone, and NAPC-ADC + 4 Gy X-ray. As expected, 100 nM ADC or 4 Gy irradiation alone showed little toxicity to 4T1-FAP and HT1080-FAP cell lines, and cell viability significantly dropped after irradiation following ADC treatment due to instantaneous release of active MMAE, indicating that MMAE released from NAPC-ADC is effective. Furthermore, FITC anti-α-tubulin antibody was applied to characterize microtubule damage under different conditions. Confocal imaging indicated that microtubule damage occurred only when treated with X-ray after incubation with NAPC-ADC (FIG. 24). ADC or X-ray treatment groups showed clear microtubule structures, whereas cells in the X-ray + ADC group rapidly shrunk due to loss of tubulin function. Thus, NAPC appears to be a promising third-generation ADC linker with good stability and high release efficiency upon X-ray irradiation in biological environments.

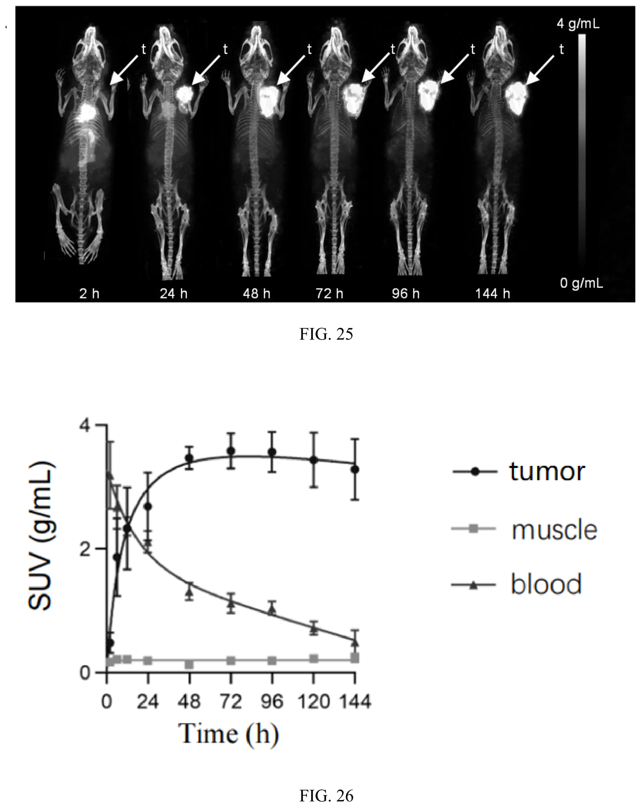

The above findings prompted the inventors to investigate the anticancer effect of radiation-activated ADC in animals. To achieve optimal therapeutic effect, correct timing of radiotherapy activation is crucial. Therefore, the inventors first studied the pharmacokinetics of NAPC-ADC via positron emission tomography-computed tomography to understand the dynamic distribution of ADCs in mice bearing 4T1-FAP xenografts. NAPC-ADC was labeled with ⁸⁹Zr radionuclide according to established protocols. After intravenous injection of [⁸⁹Zr]NAPC-ADC, 4T1-FAP tumor-bearing mice were scanned at 2, 6, 12, 24, 48, 72, 96, 120, and 144 hours. Multi-time point Positron Emission Tomography Computed Tomography (PET-CT) (FIG. 25) showed that [⁸⁹Zr]NAPC-ADC exhibited good blood circulation and high tumor uptake. Tumor uptake became visible starting at 2 hours post-injection and gradually increased until plateauing at 48 hours post-injection (~ 4 g/mL, FIG. 26). At 48 hours post-injection, radioactivity uptake in tumors was significantly higher than in blood, and tumor-to-blood ratio continued to increase as blood retention continued to decline (FIG. 26). Time-activity curves (TAC) of [⁸⁹Zr]NAPC-ADC in blood, muscle, and tumor (FIG. 26) showed that ADC accumulated at the tumor site in a time-dependent manner and reached a plateau after 48 hours, with little enrichment in blood and muscle.

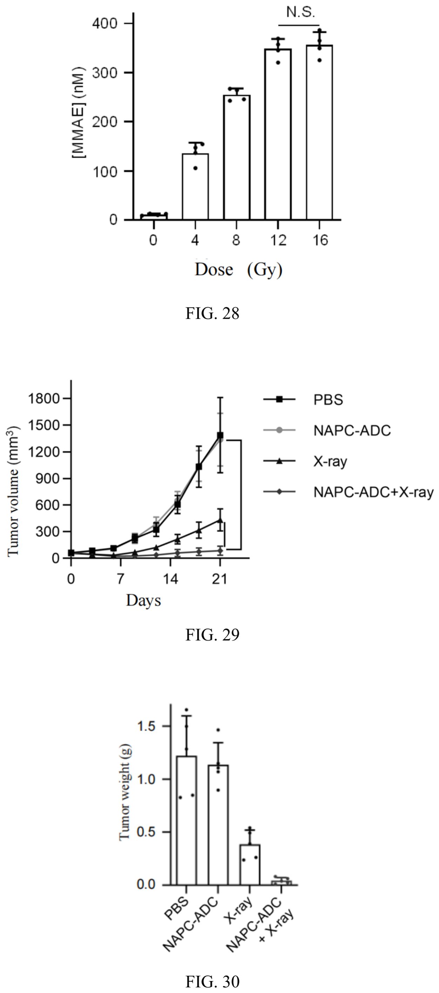

As shown in FIG. 28, 4T1-FAP tumor-bearing mice were randomly divided into 5 groups. All mice were given 5 mg/kg ADCs on day 0. Following previous *in vivo* biodistribution, X-ray was delivered at doses of 0 Gy, 4 Gy, 8 Gy, 12 Gy, and 16 Gy, and xenograft tumors were harvested, and intratumoral MMAE concentration was analyzed by LC-QTOF-MS. As desired, results showed that 4T1-FAP tumors treated with NAPC-ADC released MMAE in a dose-dependent manner after radiotherapy, indicating that MMAE release is dependent on radiation-induced reduction reactions. When irradiated with 12 Gy radiation, 100 nM NAPC-ADC could completely release MMAE, with a G-value for MMAE in tumors of 35 nM/Gy. The success of the radiation-induced controlled release strategy using antibody-drug delivery systems is expected to enhance therapeutic efficacy.

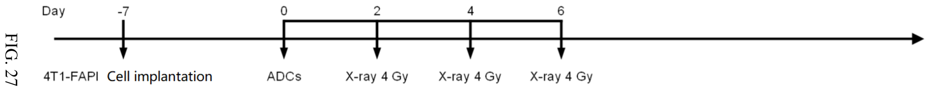

Subsequently, the effect of radiation-induced payload release was evaluated. Tumor-bearing mice with 40-70 mm³ tumors were randomly divided into 4 groups, treated with PBS, NAPC-ADC, X-ray, and NAPC-ADC + X-ray, respectively. All mice were given 5 mg/kg ADCs on day 0. For treatment groups, based on 12 Gy X-ray triggering complete MMAE release (FIG. 27), 4 Gy X-ray was administered on days 2, 4, and 6 after ADC injection. NAPC-MMAE combined with 3×4 Gy X-ray showed significant efficacy against 4T1-FAP tumors. As shown in FIG. 29, by day 21 only, the average tumor size was about 100 mm³, whereas the control group (PBS only) reached 1500 mm³ tumor size (FIG. 29), and administration of NAPC-ADC alone at 5 mg/kg concentration had no significant effect on tumor growth, indicating that the observed therapeutic response is specific to radiation-induced payload release *in vivo.* Thus, combination therapy of X-ray and NAPC-ADC significantly inhibited tumor growth (FIG. 29), while NAPC-ADC treatment could hardly halt tumor growth, and X-ray inhibited its growth in a limited manner. Tumor-bearing mice were euthanized on day 21, tumors were harvested for tumor weight measurement and photography (FIG. 30), confirming the therapeutic effect of NAPC-ADC + radiotherapy. A significant difference existed between the treatment group and control groups (P<0.0001). Body weight assessment over 21 days (FIG. 31) and histological analysis of key organs indicated that the prodrug showed no overall toxicity, suggesting satisfactory biosafety of the inventors' strategy.

The results demonstrate that radiation-induced controlled release strategy holds promise for treating tumors with limited toxicity. It also reveals another advantage of radiation reduction reactions for *in vivo* controlled release: a dual lock on tumor for prodrug activation. On one hand, radiotherapy is precisely applied for spatial localization of tumors. On the other hand, e⁻_{aq} is quenched by high levels of oxygen in normal tissues, rejecting drug release, providing additional selectivity for hypoxic tumors to improve the efficiency of radiotherapy-induced prodrug activation.

## Claims

1. A radiation-activatable compound, or a pharmaceutically acceptable salt, or solvate, or stereoisomer thereof, wherein the compound has a structure of formula (I) or formula (II): 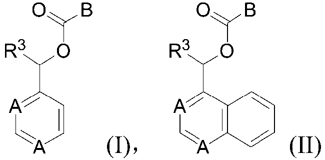 **characterized in that**:
A is CR¹, N or NR², and only one A in Formula (I) is N or NR², only one A in Formula (II) is N or NR², the carbon atom in the aromatic ring in Formula (I) or Formula (II) is optionally substituted with R¹, wherein R¹ is selected from the group consisting of -H and electron-withdrawing groups, preferably selected from the group consisting of -H, -NO₂, -CN, and halogen, more preferably selected from the group consisting of -H, -NO₂, -CN, and -F;
when A is NR², R² is alkyl, preferably C₁₋₆ alkyl, more preferably C₁₋₃ alkyl; R² is optionally substituted with a linking group comprising one or more groups that are or are derived from the group consisting of amino, amido, heterocyclyl, alkyl, alkenyl, alkynyl, polyethylene glycol (PEG), amino acid;
B is any therapeutic agent or chromogenic agent capable of coupling with the aryl ester group moiety of the compound, provided that it can be separated from the aryl ester group moiety under radiation action and release a molecule with therapeutic activity or chromogenic property, for example, B is a fluorescent group, or a drug group, preferably a drug group for treating cancer, and more preferably a chemotherapeutic drug group;
R³ is selected from the group consisting of -H and electron-donating groups, preferably selected from the group consisting of -H, alkyl and aryl, more preferably selected from the group consisting of -H, C₁₋₆ alkyl and C₆₋₁₀ aryl, wherein said alkyl and aryl are optionally substituted.

2. The radiation-activatable compound according to claim 1, or a pharmaceutically acceptable salt, or solvate, or stereoisomer thereof, wherein the compound has a structure of one of the following formulas: 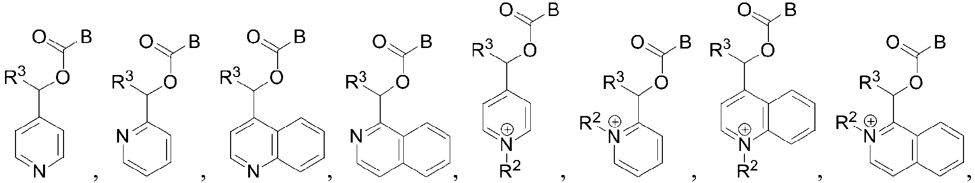
wherein carbon atoms in the aromatic ring are optionally substituted with R¹; R¹, R², R³ and B have meanings as decribed in claim 1,
preferably in the simultaneous presence with water, after radiation, the aryl ester group in Formula (I) or Formula (II) can be removed, thereby releasing B.

3. The radiation-activatable compound according to claim 1 or claim 2, or a pharmaceutically acceptable salt, or solvate, or stereoisomer thereof, wherein
R¹ are all -H,
R², if present, is methyl, which is optionally substituted with a linking group, and/or
R³ is -H,
the linking group has the meaning as described in claim 1.

4. The radiation-activatable compound according to claim 1 or claim 2, or a pharmaceutically acceptable salt, or solvate, or stereoisomer thereof, wherein
one or more R¹ in Formula (I) or Formula (II) is selected from the group consisting of -NO₂, -CN, and -F,
R², if present, is methyl, and/or
R³ is -H.

5. The radiation-activatable compound according to claim 1 or claim 2, or a pharmaceutically acceptable salt, or solvate, or stereoisomer thereof, wherein
R¹ are all -H,
R², if present, is methyl, and/or
R³ is C₁₋₃ alkyl or phenyl.

6. The radiation-activatable compound according to any one of claims 1 to 5, or a pharmaceutically acceptable salt, or solvate, or stereoisomer thereof, wherein said B is coumarin and derivatives thereof, rhodamine and derivatives thereof, or auristatin and derivatives thereof.

7. The radiation-activatable compound according to any one of claims 1 to 6, or a pharmaceutically acceptable salt, or solvate, or stereoisomer thereof, wherein said B is selected from the group consisting of the following structures: 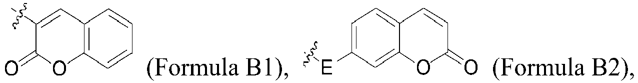 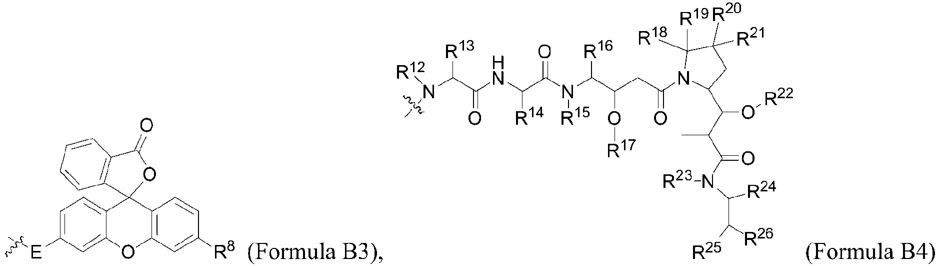
wherein 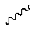 indicates the site where said B attached to the ester group in Formula (I) or Formula (II); the ring carbon atoms in the Formula (B1), Formula (B2) and Formula (B3) can be optionally substituted by -R⁵, -NR⁶R⁷;
E is an NR⁴ group or O, preferably an NR⁴ group;
R⁸ is NR⁹R¹⁰ or OR⁹;
when said B is auristatin and derivatives thereof, said B is attached to the ester group in Formula (I) or Formula (II) via NR⁴;
R⁴ is -H or alkyl, preferably -H or C₁₋₆ alkyl, more preferably -H or C₁₋₄ alkyl;
each of R⁵, R⁶, R⁷, R⁹ and R¹⁰ is independently selected from H and alkyl, and said alkyl is preferably C₁₋₆ alkyl, more preferably C₁₋₄ alkyl;
in Formula (B4), each of R¹² to R²³ is independently selected from H and alkyl, and said alkyl is preferably C₁₋₆ alkyl, more preferably C₁₋₄ alkyl;
R²⁴ is selected from H, hydroxyl, carboxyl, alkyl, alkoxy, halogen, aryl or heteroaryl, said aryl is preferably C₆₋₁₀ aryl, and said heteroaryl is preferably C₅₋₁₀ heteroaryl;
R²⁵ is selected from H, hydroxyl, alkyl, alkoxy, and halogen;
R²⁶ is selected from aryl or heteroaryl, said aryl or heteroaryl is optionally further substituted by a substituent selected from H, halogen, hydroxy, alkyl, and alkoxy, said aryl is preferably C₆₋₁₀ aryl, said heteroaryl is preferably C₅₋₁₀ heteroaryl.

8. The radiation-activatable compound according to any one of claims 1 to 7, or a pharmaceutically acceptable salt, or solvate, or stereoisomer thereof, wherein said B is selected from the group consisting of the following structures: 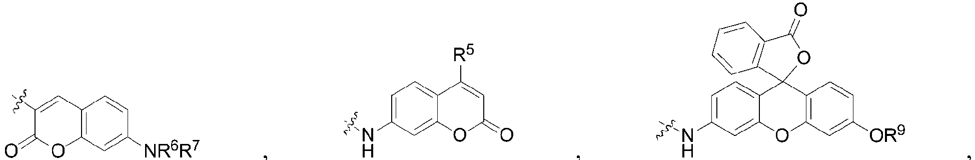 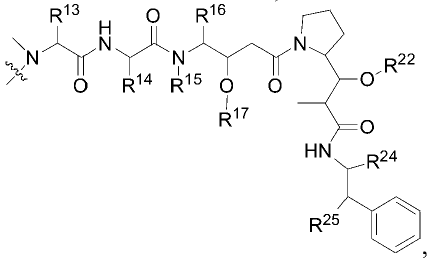
wherein each of R⁵, R⁶, R⁷, R⁹, R¹³ to R¹⁷, and R²² is independently C₁₋₆ alkyl, more preferably C₁₋₄ alkyl, more preferably R⁶ and Rare ethyl, R⁵ and R⁹ are methyl;
R²⁴ is selected from H, hydroxyl, carboxyl, C₁₋₆ alkyl, C₁₋₆ alkoxy, halogen, C₆₋₁₀ aryl and C₅₋₁₀ heteroaryl;
R²⁵ is selected from H, hydroxy, and C₁₋₆ alkyl;
the radiation-activatable compound is preferably selected from the group consisting of: 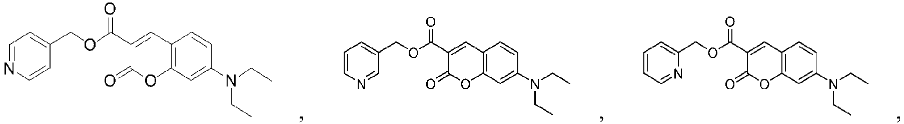 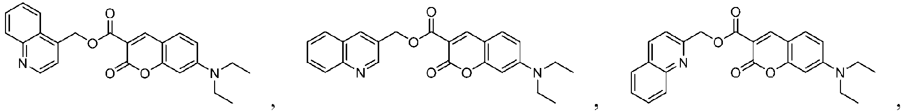 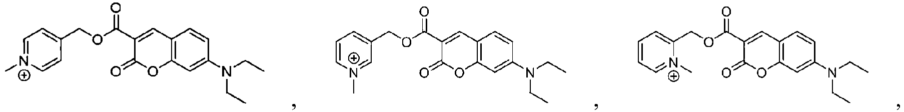 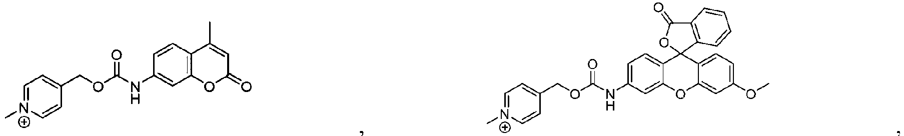 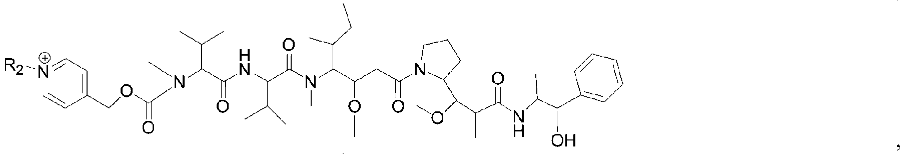 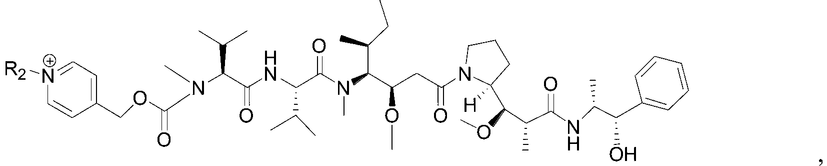 and 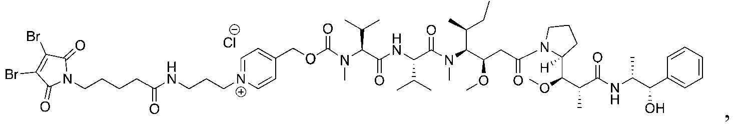 wherein R² is C₁₋₆ alkyl.

9. Use of the radiation-activatable compound according to any one of claims 1 to 8, or a pharmaceutically acceptable salt, or solvate, or stereoisomer thereof, in preparing a drug conjugate, **characterized in that**, in Formula (I) or Formula (II), only one A is NR², R² is alkyl, preferably C₁₋₆ alkyl, more preferably C₁₋₃ alkyl; R² is optionally substituted by a linking group comprising one or more groups that are or are derived from amino, amido, heterocyclyl, alkyl, alkenyl, alkynyl, polyethylene glycol (PEG), amino acid.

10. The use according to claim 9, wherein a drug moiety is attached to Formula (I) or Formula (II) via R², or by reacting with R², thereby preparing the drug conjugate.

11. A drug conjugate, or a pharmaceutically acceptable salt, or solvate, or stereoisomer thereof, the drug conjugate comprises a targeting moiety, a linking moiety and a drug moiety, wherein the linking moiety couples the targeting moiety and the drug moiety, and wherein the linking moiety comprises a structure of Formula (I') or Formula (II'): 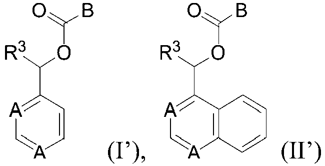 **characterized in that**:
A is CR¹ or NR^{2'}, and only one A in Formula (I') is NR^{2'}, only one A in Formula (II') is NR^{2'}, the carbon atom in the aromatic ring in Formula (I') or Formula (II') is optionally substituted with R¹, wherein R¹ is selected from the group consisting of -H and electron-withdrawing groups, preferably selected from the group consisting of -H, -NO₂, -CN, and halogen, more preferably selected from the group consisting of -H, -NO₂, -CN, and -F;
R^{2'} is alkyl, preferably C₁₋₆ alkyl, more preferably C₁₋₃ alkyl; R^{2'} is substituted with a linking group attached to said targeting moiety;
B is a fluorescent group, or a drug group, preferably a chemotherapeutic drug group;
R³ is selected from the group consisting of -H and electron-donating groups, preferably selected from the group consisting of -H, alkyl and aryl, more preferably selected from the group consisting of -H, C₁₋₆ alkyl and C₆₋₁₀ aryl, and said alkyl and aryl are optionally substituted;
said targeting moiety is an antibody.

12. The drug conjugate according to claim 11, or a pharmaceutically acceptable salt, or solvate, or stereoisomer thereof, the structure of Formula (I') or Formula (II') having a structure of one of the following formulas: 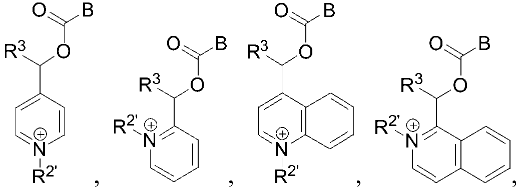 wherein carbon atoms in the aromatic ring are optionally substituted with R¹; R¹, R^{2'}, R³ and B have meanings as decribed in claim 1.

13. The drug conjugate according to claim 11 or 12, or a pharmaceutically acceptable salt, or solvate, or stereoisomer thereof, wherein
R¹ are all -H, and/or
R³ is -H.

14. The drug conjugate according to claim 11 or 12, or a pharmaceutically acceptable salt, or solvate, or stereoisomer thereof, wherein
one or more R¹ in Formula (I) or Formula (II) is selected from the group consisting of -NO₂, -CN, and -F, and/or
R³ is -H.

15. The drug conjugate according to claim 11 or 12, or a pharmaceutically acceptable salt, or solvate, or stereoisomer thereof, wherein
R¹ are all -H, and/or
R³ is C₁₋₃ alkyl or phenyl.

16. The drug conjugate according to any one of claims 11 to 15, or a pharmaceutically acceptable salt, or solvate, or stereoisomer thereof, wherein the linking group comprises one or more groups that are or are derived from amino, amido, heterocyclyl, alkyl, alkenyl, alkynyl, polyethylene glycol (PEG), amino acid.

17. A method for activating a radiation-activatable compound or a drug conjugate with radiation, **characterized in that** the radiation-activatable compound or drug conjugate is the compound according to any one of claims 1 to 8 or the drug conjugate according to any one of claims 11 to 16, or a pharmaceutically acceptable salt, or solvate, or stereoisomer thereof.

18. The method according to claim 17, wherein the radiation is a high-energy radiation ray, preferably X-rays or gamma rays, more preferably the intensity of said X-rays or gamma rays is 60 Gy or less, 50 Gy or less, 40 Gy or less, 30 Gy or less, 20 Gy or less, 10 Gy or less, 8 Gy or less, 6 Gy or less, and 4 Gy or less.

19. A method for treating or diagnosing a disease, the method comprising administering to an individual a radiation-activatable compound or a drug conjugate, and irradiating the individual with a therapeutically effective amount of radiation, the radiation-activatable compound or the drug conjugate is the compound according to any one of claims 1 to 8 or the drug conjugate according to any one of claims 11 to 16, or a pharmaceutically acceptable salt, or solvate, or stereoisomer thereof.

20. The method according to claim 19, the method is used for treating or inhibiting cancer, reducing its severity, reducing its risk, or inhibiting its metastasis in an individual.

21. The method according to claim 19 or 20, wherein the cancer is selected from breast cancer, colorectal cancer, lung cancer and gastric cancer.

22. The method according to any one of claims 19 to 21, wherein the individual is irradiated after administration of the compound or the drug conjugate, preferably 24 hours, 48 hours, 96 hours, and/or 144 hours after administration of the compound or the drug conjugate.

23. The method according to any one of claims 19 to 22, wherein the compound or the drug conjugate is administered in an amount from about 0.005 mg/kg to about 100 mg/kg, for example, a dose of about 0.005 mg/kg, 0.05 mg/kg, 0.5 mg/kg, 5 mg/kg, 10 mg/kg, 20 mg/kg, 30 mg/kg, 40 mg/kg, 50 mg/kg, 100 mg/kg.

24. The method according to any one of claims 19 to 23, wherein the compound or the drug conjugate, and the radiation are administered once per week, once every two weeks, once every three weeks, or once every four weeks, and the compound or the drug conjugate, and the radiation are administered continuously for at least 1 round, at least 2 rounds, at least 3 rounds, at least 4 rounds, at least 5 rounds, at least 6 rounds, at least 7 rounds, at least 8 rounds, at least 9 rounds, at least 10 rounds.

25. The method according to any one of claims 19 to 24, wherein the compound or the drug conjugate is administered parenterally, for example, administered intravenously.

26. The method according to any one of claims 19 to 25, wherein the radiation is a high-energy radiation ray, preferably X-rays or gamma rays, more preferably the intensity of said X-rays or gamma rays is 60 Gy or less, 50 Gy or less, 40 Gy or less, 30 Gy or less, 20 Gy or less, 10 Gy or less, 8 Gy or less, 6 Gy or less, and 4 Gy or less.
